# EUROPEAN PATENT APPLICATION

(11) **EP 4 417 623 A1**
(43) Date of publication of application: **21.08.2024**
(21) Application number: 23382147.9
(22) Date of filing: 15.02.2023
(51) Int. Cl.: C07K 16/28, A61K 39/00, A61P 35/02

(54) **CCR9 TARGETING MOIETY FOR THE TREATMENT OF CCR9-POSITIVE CANCER**

(71) Applicant: Fundació Institut de Recerca Contra la Leucèmia Josep Carreras, 08916 Barcelona (ES); Institució Catalana de Recerca I Estudis Avançats, 08010 Barcelona (ES); Onechain Immunotherapeutics SL, 08021 Barcelona (ES); Fundació Institut d'Investigació en Ciències de la Salut Germans Trias i Pujol, 08916 Badalona, Barcelona (ES)
(72) Inventor: MENÉNDEZ BUJÁN, Pablo, 08916 Badalona (ES); SÁNCHEZ MARTÍNEZ, Diego, 08916 Badalona (ES); TIRADO CABRERA, Néstor, 08916 Badalona (ES); FERNÁNDEZ FUENTES, Narciso, 08916 Badalona (ES); DÍAZ CORTÉS, Víctor Manuel, 08021 Barcelona (ES)
(74) Representative: Hoffmann Eitle

(57) **Abstract**

The present invention provides therapeutics for the treatment of CCR9-positive cancers such as T-cell acute lymphoblastic leukemia. In particular, the present invention provides a CCR9 targeting moiety. The present invention furthermore relates to a CCR9 targeting moiety comprising a further targeting moiety, preferably a CD1a targeting moiety.

## Description

### Technical Field

The present invention provides therapeutics for the treatment of CCR9-positive cancers such as T-cell acute lymphoblastic leukemia. In particular, the present invention provides a CCR9 targeting moiety. The present invention furthermore relates to a CCR9 targeting moiety comprising a further targeting moiety, preferably a CD1a targeting moiety.

### Background art

T-cells are major effectors in cancer immunotherapy¹. Infusion of unmodified donor- or third-party-derived T-cells can be used to control resistant disease in patients who have received allogeneic hematopoietic stem cell transplantation (alloHSCT)². The effector functions of T-cells can be substantially boosted by redirecting them against tumor antigens *via* genetic engineering of T-cell receptors or CARs to generate tumor-reactive T-cells for adoptive transfer³. CARs are fusion molecules typically containing an extracellular single chain variable fragment (scFv) of a monoclonal antibody specific to a surface molecule on the tumor cell, a spacer domain that provides flexibility and optimizes effector-target cell engagement, a transmembrane domain, and the T-cell signaling domains 4-1 BB (or CD28) and CD3ζ, which drive survival/proliferation and cytotoxicity, respectively^{4,5}. CAR T-cell (CART) therapy has revolutionized cancer treatment, especially for relapse/refractory (R/R) B-cell acute lymphoblastic leukemia (B-ALL) and B-cell lymphomas, for which a specific and relatively safe target antigen exists (CD19)⁶. Indeed, impressive rates of clinical complete response (>85%) have been independently reported in patients with R/R B-ALL receiving CD19-directed CARTs⁷.

However, CART therapies are still in their infancy, and our current understanding of the mechanisms underlying successful adoptive immunotherapy and the clinic-biological parameters predicting response to immunotherapy remain elusive. Of note, CART therapies for non-B-cell malignancies are lagging owing to the absence of safe and specific targets, and/or CART delivery-associated challenges.

T-ALL is a phenotypically and genetically heterogeneous malignant disorder that results from leukemic transformation of thymic T-cell precursors⁸. It comprises 10-15% of all acute leukemias diagnosed in children and adults^{9,10}. Despite improved survival rates thanks to intensive chemotherapy regimens, event-free (EFS) and overall (OS) survival remains <70%, and R/R T-ALL has a particularly poor outcome. There are currently no potential curative options for R/R T-ALL beyond HSCT and conventional chemotherapy, which is linked to large trade-offs in toxicities^{9,11}, bolstering the need for novel targeted therapies. Strategies targeting T-cell malignancies using any kind of immunotherapy (including CARTs) remain challenging because of the shared expression of target antigens between normal and malignant T-cells, ultimately leading to life-threatening T-cell aplasia and fratricide of CARTs, which limits their therapeutic efficacy¹²⁻¹⁸. Several clinical trials in the last few years have explored the therapeutic use of CD5- and CD7-directed CARTs for T-ALL with encouraging results¹⁹⁻²¹. However, CD7-directed CART therapy will likely induce both immunodeficiency and CAR T-cell fratricide because of the shared expression of CD7 in normal and tumoral T-cells, and it may only be useful as a bridge to allo-HSCT. Although allogenic CART therapies using gene-edited T cells depleted of CD7, TRAC, CD52 or HLA-II represent innovative strategies²²⁻²⁵, improved efficacious autologous CARTs with a safe profile are in high demand for T-ALL.

Cortical T-ALL (coT-ALL) is a major subgroup of T-ALL characterized by the cell surface expression of CD1a, a lipid-presenting molecule whose expression is essentially restricted to coT-ALL and Langerhans cell histiocytosis. CD1a is not expressed in any human tissue (completely absent in normal mature T-cells) with the exception of cortical thymocytes and Langerhans cells during development^{26,27}. The feasibility of fratricide-resistant CD1a-specific CARTs as an *unprecedented* adoptive immunotherapy strategy for coT-ALL, which showed robust cytotoxicity against CD1a+ T-ALL cell lines and primary coT-ALL blasts both *in vitro* and *in vivo* has been reported. CAR-CD1a T-cells are fratricide-resistant and remain functional *in vivo* after 8 weeks, as demonstrated in leukemia re-challenge experiments²⁸.

CCR9 is a 7-transmembrane protein chemokine receptor whose unique ligand is CCL25²⁹. High expression of CCR9 is associated with poor prognosis or metastasis in different types of solid tumors³⁰⁻³³. Antibodies against CCR9 have recently demonstrated antileukemic effects in preclinical models of T-cell leukemia³⁴. Importantly, a CCR9-directed CAR has very recently been proposed for R/R T-ALL³⁵.

The present invention aims to provide a novel therapy for treating T-ALL.

### Figures

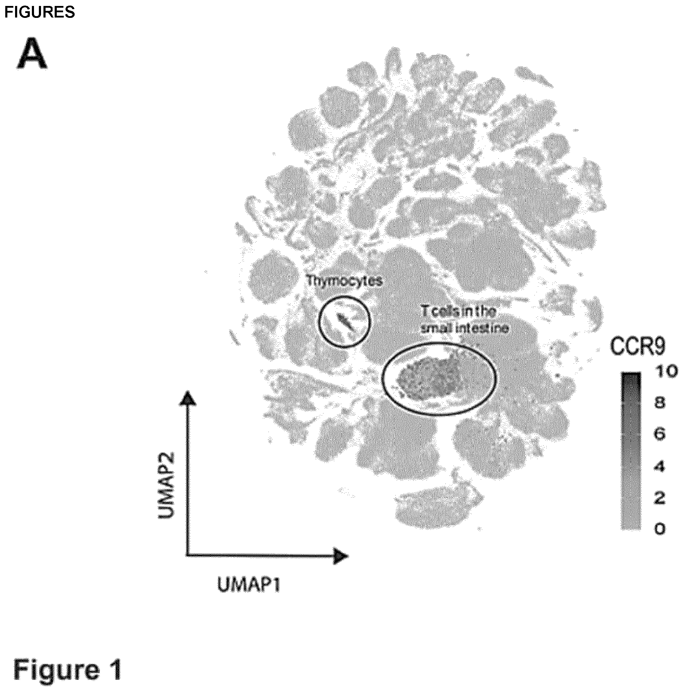

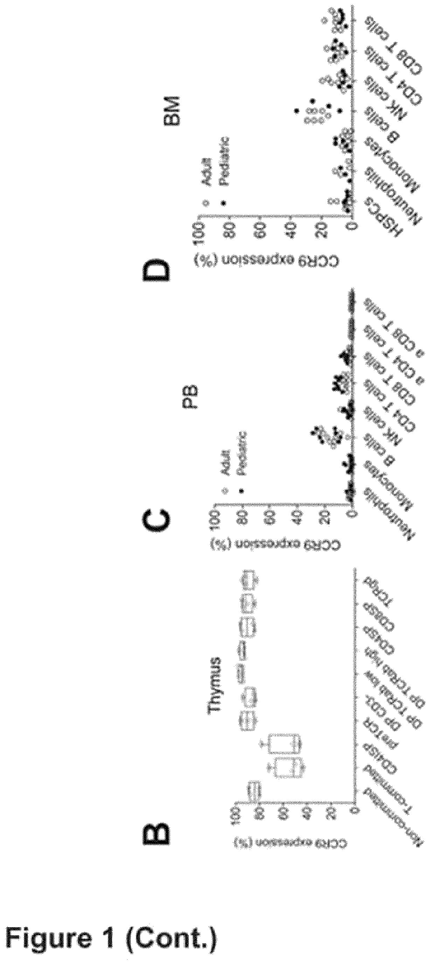

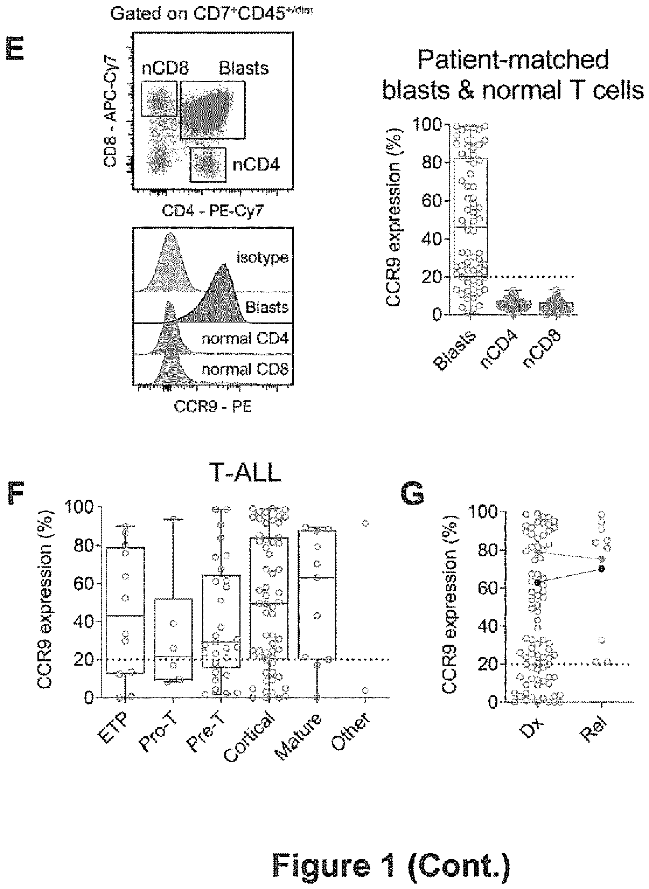

Figure 1. CCR9 expression in healthy tissues and T-ALL samples. A) Single-cell RNA seq analysis of CCR9 expression in healthy tissues (Tabula Sapiens Consortium, Science (2022)). Two-dimensional clustering analysis on Uniform Manifold Approximation and Projection (UMAP) to visualize CCR9 expression on Thymocytes and T cells; B) CCR9 expression in human neonatal thymus subpopulations determined by FACS (n=4). C) & D) CCR9 expression in human peripheral blood (PB, n=18) and bone marrow (BM, n=13) respectively, from adult and pediatric healthy donors. E) Gating strategy, representative histogram, and total CCR9 expression in blasts and normal CD4 (nCD4) and CD8 (nCD8) T cells from T-ALL patients (n=66). F) CCR9 expression in blasts from T-ALL patients stratified across immunophenotypes/subtypes (n=120). ETP, Early T-cell precursor (ETP) acute lymphoblastic leukemia. G) CCR9 expression in T-ALL blasts at diagnosis (Dx) and relapse (Rel), two patient-matched samples are shown.

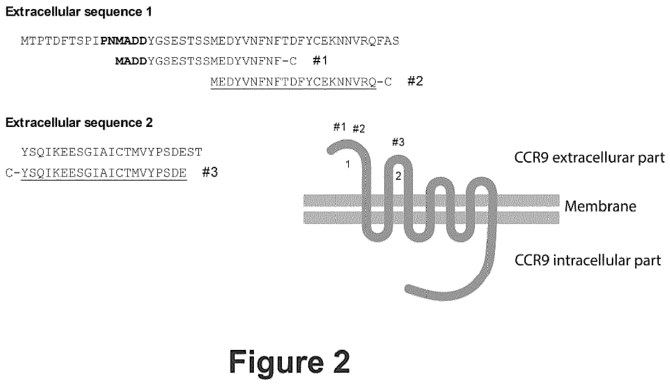

Figure 2. CCR9 antibody generation strategy. Anti-CCR9 antibody-secreting hybridomas were generated by mouse immunisation with human CCR9 peptides. Extracellular sequences/regions of CCR9 that were chosen for mouse immunisation. From extracellular sequence 1 two different 21-aminoacid long peptides were used (#1 and #2). From extracellular sequence 2 one peptide was used (#3). PNMADD peptide in extracellular sequence 1 denotes the epitope that is recognized by the prior art anti-CCR9 antibody clones 92R and 91R (SunRock, Somovilla-Crespo et al, Front Immunol (2018) and PCT/EP2014/075578). Lower panel, CCR9 transmembrane domains and location of extracellular sequences 1 and 2 and peptides #1, #2 and #3.

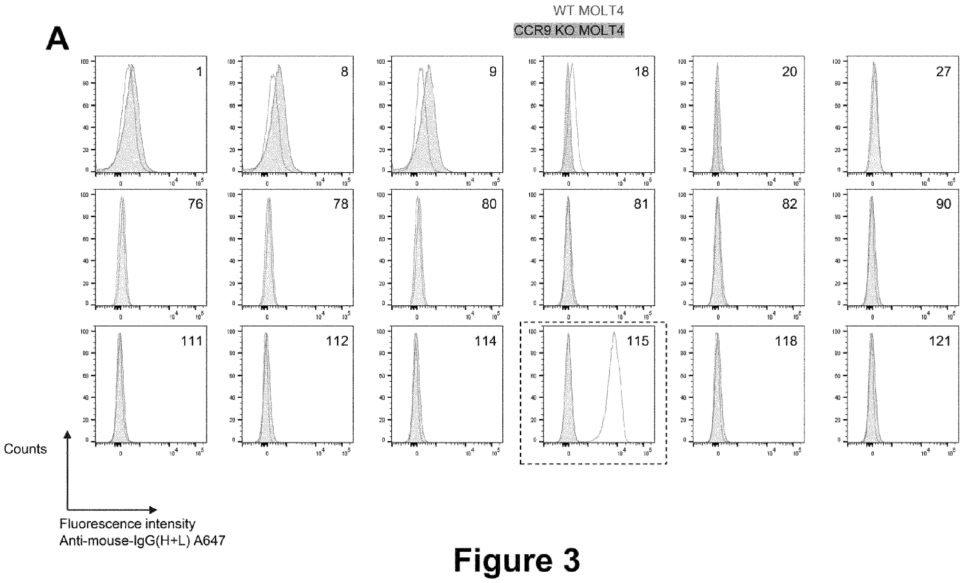

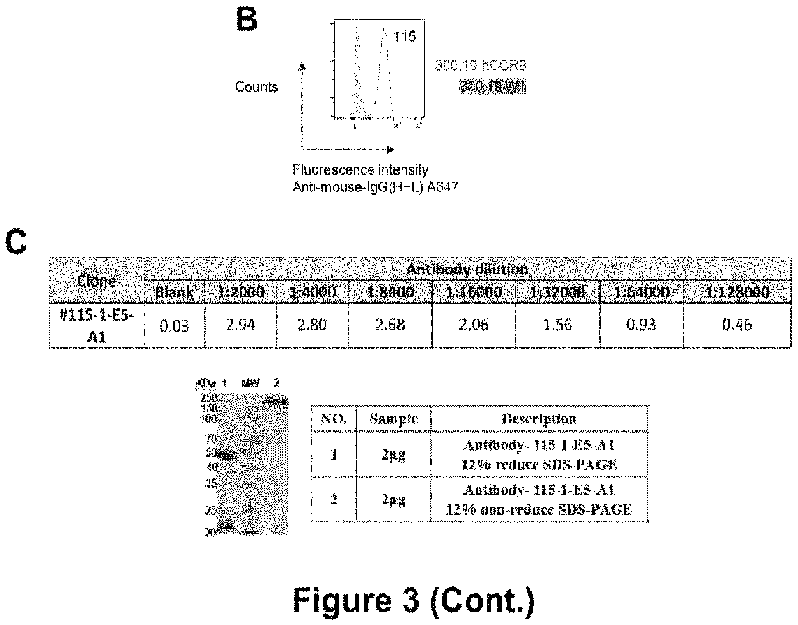

Figure 3. Screening of Hybridoma clones via flow cytometry. A total of 82 hybridoma (hyb) candidates were tested by FACS analysis (MADDY peptide: 40 clones; MEDY peptide: 6 clones; YSQIK peptide: 36 clones) as further described in Example 2. A) The figure shows histogram analysis of exemplary results of 18 representative tests in WT and knock-out (KO) for CCR9 MOLT4 cells. Only one hybridoma clone of the 82 tested positive by flow cytometry in WT cells but not in CCR9 KO cells (Clone 115, dashed square). B) Clone 115 was further tested using the CCR9-negative 300.19 cell line (WT) and its CCR9-overexpressing counterpart. C) Clone 115 titer determined by ELISA (upper panel) and SDS-PAGE followed by Coomassie staining quality control analysis (lower panel) under reducing (line 1) and non-reducing conditions (line 2). MW, molecular weight marker. Antibody dilutions used for ELISA titer determination (upper) and sample amount loaded in SDS-PAGE (lower) are indicated.

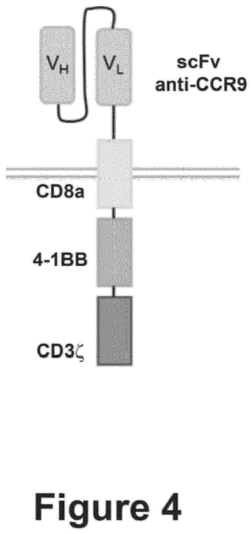

Figure 4. Structure of one chimeric antigen receptor (CAR) of CCR9 of present invention

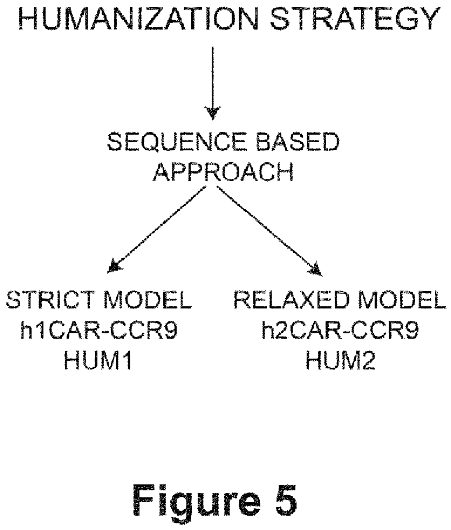

Figure 5. CCR9 scFv humanization strategy. Summary scheme of humanization strategies followed to obtain the different humanization constructs h1CAR-CCR9 (HUM1) and h2CAR-CCR9 (HUM2).

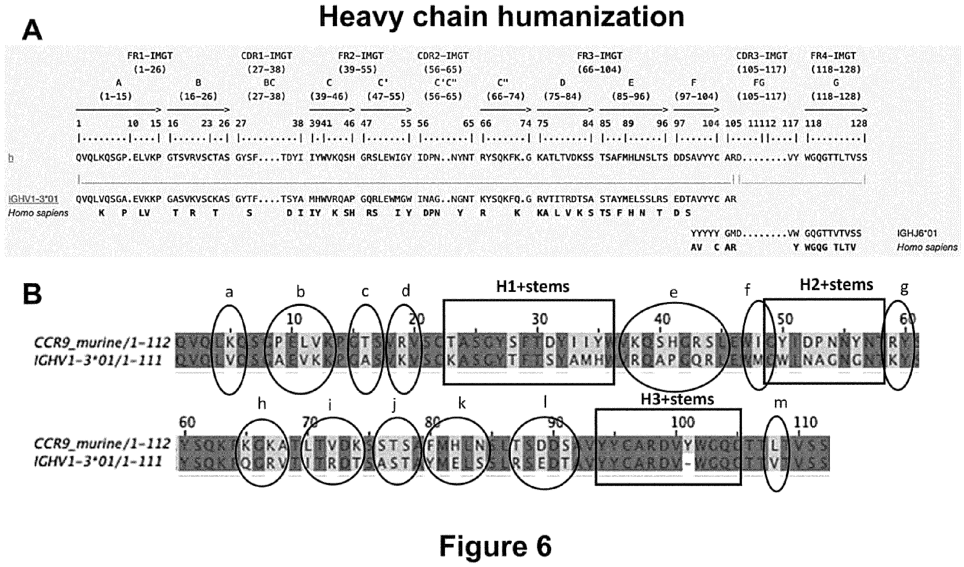

Figure 6. Sequence-based humanization. A) Alignment between the murine heavy chain and the germline IGHV1-3*01 sequence that shares the highest sequence identity to the heavy chain (60.2%). The number of different residues (with different levels of conservation) without considering CDRs is 32. B) Comparison between the murine and IGHV1-3*01 sequence. Letters and circles indicate aminoacid changes introduced (see Examples section for a detailed explanation). CDRs and stems are indicated for the heavy chain (H1 to H3).

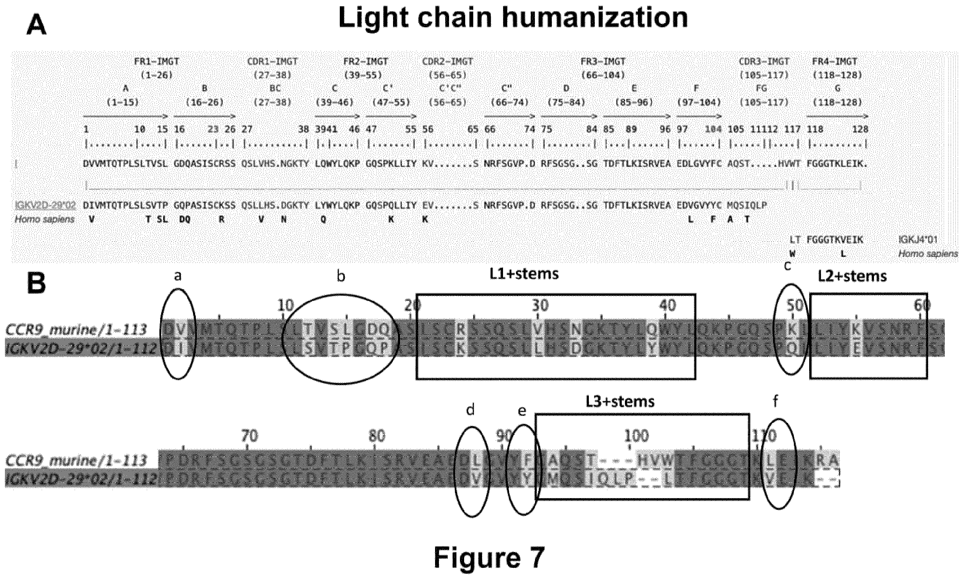

Figure 7. Sequence-based humanization. A) Alignment between the murine light chain and the germline IGKV2D-29*02 that shares the highest sequence identity to the light chain (82%). The number of different residues without considering CDRs is 13. B) Comparison between the murine and IGKV2D-29*02 sequence. Letters and circles indicate aminoacid changes introduced (see Examples section for a detailed explanation). CDRs and stems are indicated for the heavy light chains (L1 to L3).

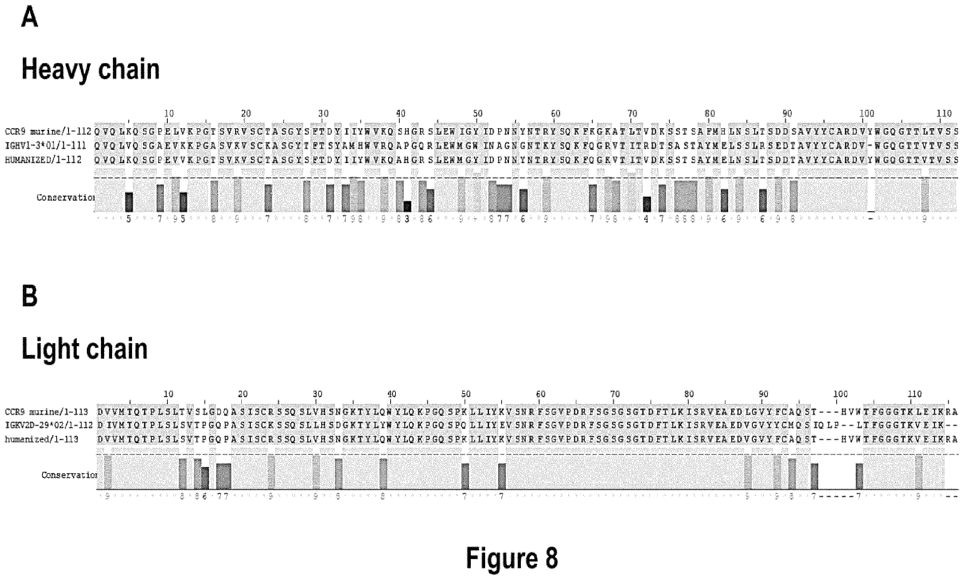

Figure 8. Sequence-based humanization. Alignment of murine, germline and proposed humanized sequences, showing the conservation degree for both heavy (upper panel) and light (lower panel) chains.

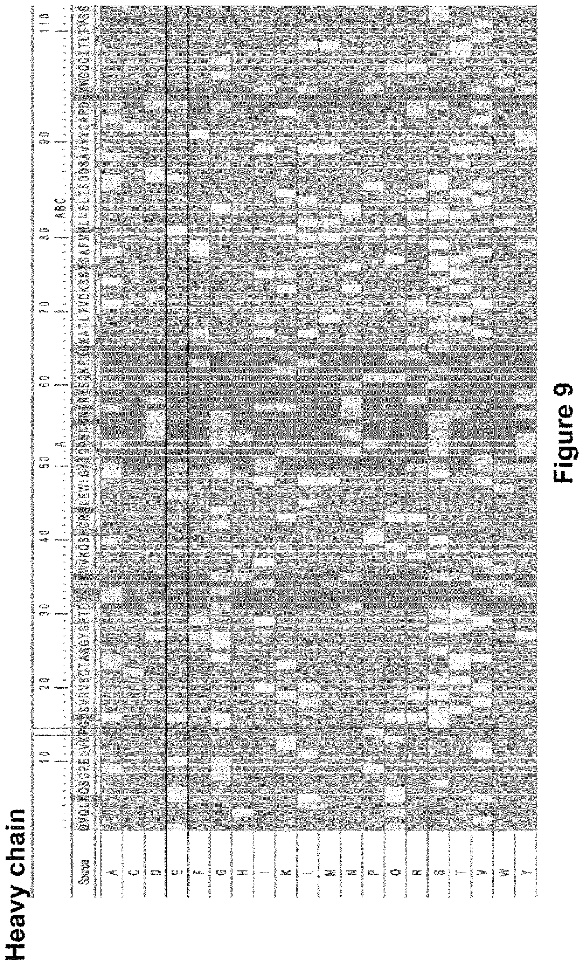

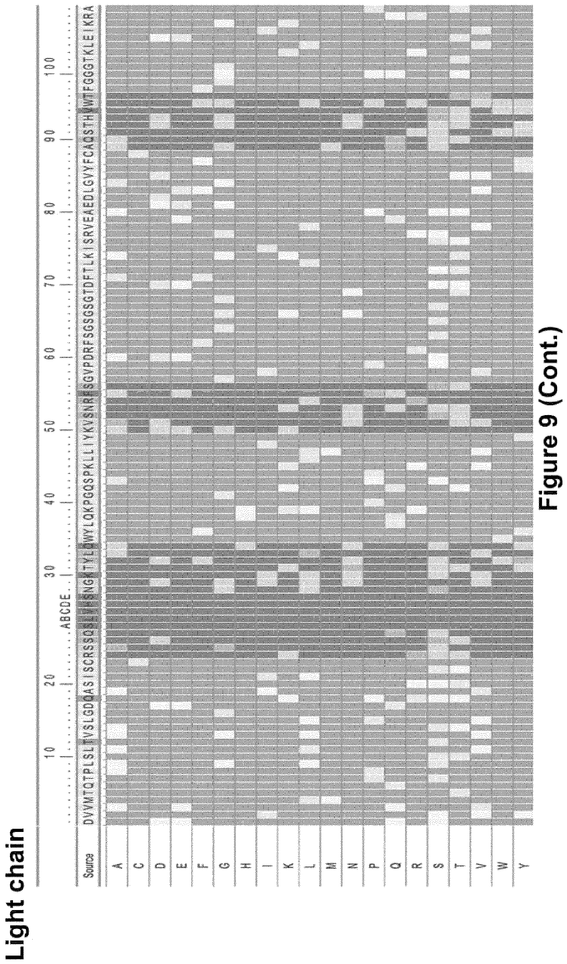

Figure 9. Sequence-based humanization. Heavy and light chain conservation among the different human sequences.

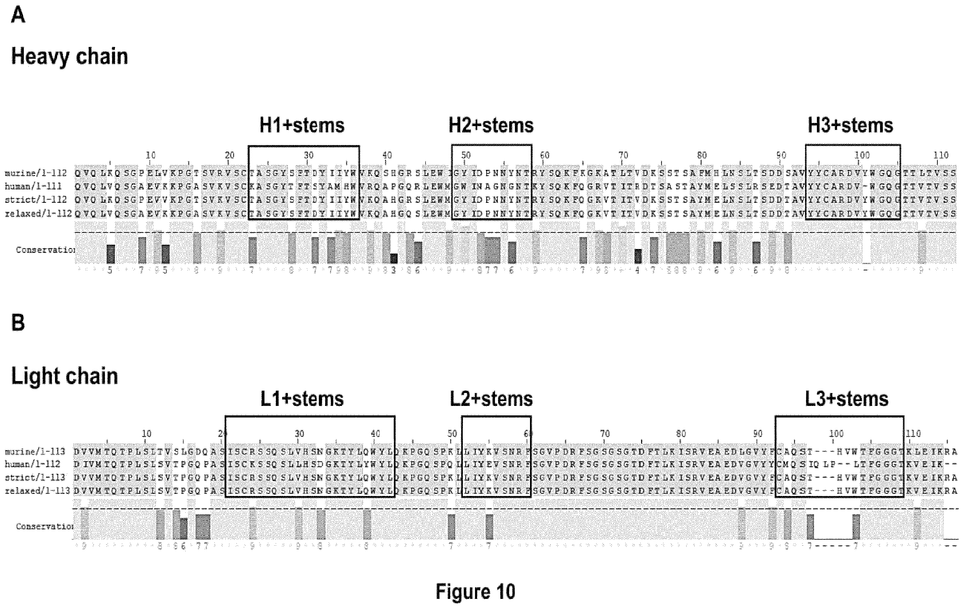

Figure 10. Sequence-based humanization. Sequence comparison between murine, human germline, and humanized sequence-based "strict" (minimum changes were introduced) and sequence-based "relaxed" (more changes were allowed) candidates, for the heavy chain (upper panel) and for the light chain (lower panel). CDRs and stems are indicated for the heavy (H1 to H3) and light chains (L1 to L3).

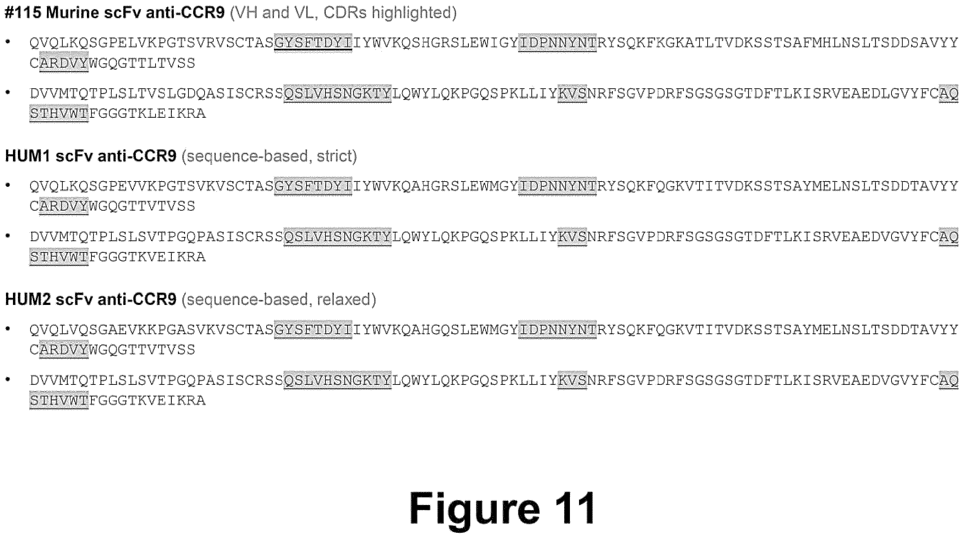

Figure 11. Sequence-based humanization. Sequences of the VH and VL chains of the scFvs of murine clone 115 ("murine scFv") as well as the two humanized scFvs ("HUM1 scFv" and HUM2 scFv") anti-CCR9. The CDRs are highlighted.

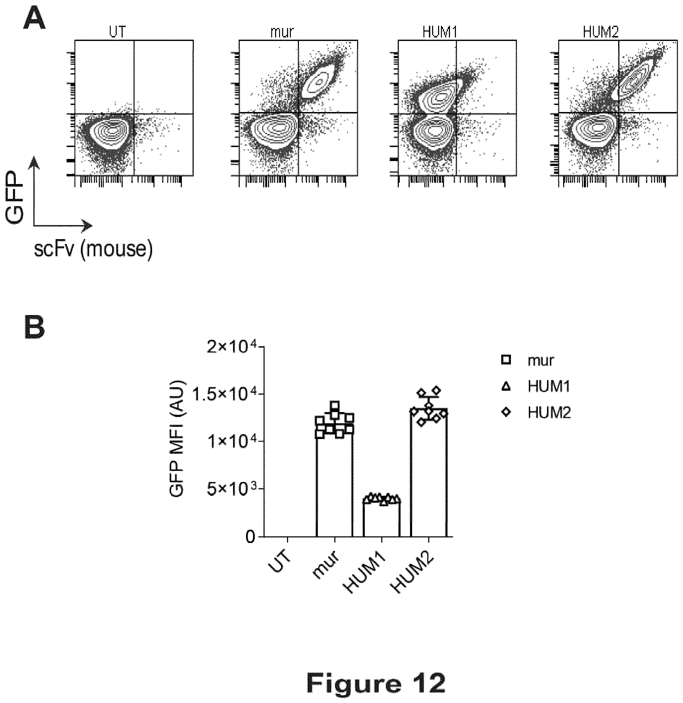

Figure 12. CCR9 detection in T cells. A) Representative flow cytometry plots showing CAR construct detection in transduced T cells (GFP+); (mur: murine CAR; HUM1 CAR-CCR9 and HUM2 CAR-CCR9). B) Median fluorescence intensity (MFI) of GFP in the transduced populations.

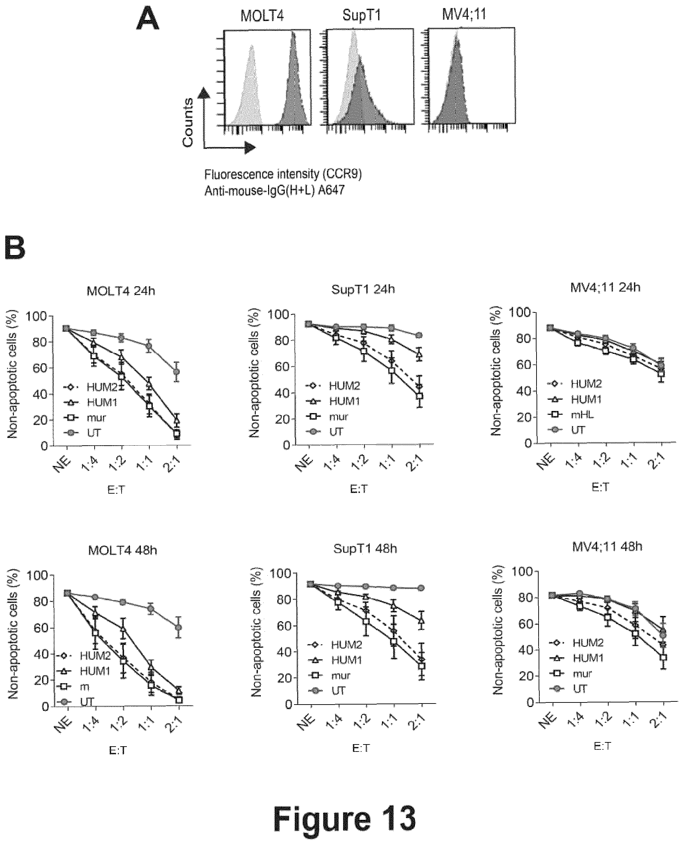

Figure 13. CAR-CCR9 in vitro cytotoxicity. A) CCR9 expression in indicated cells lines analyzed by flow cytometry with high expression (MOLT4), median expression (SupT1) and no expression (MV4;11). B) Effector T cells were co-cultured with the selected target cell lines at different effector target ratios (E:T) for 24h (top) and 48h (bottom), and residual non-apoptotic target cells were measured by flow cytometry (n=5 donors).

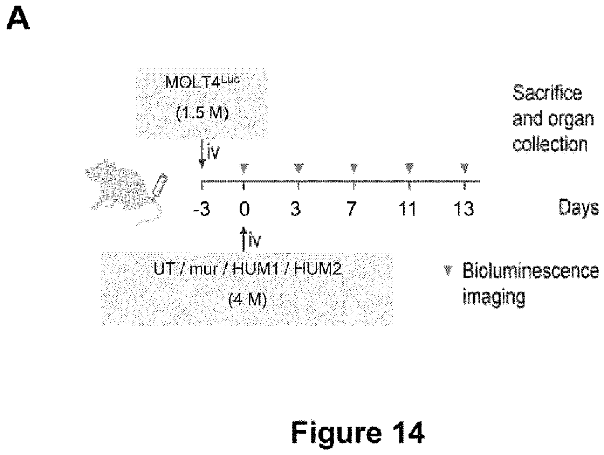

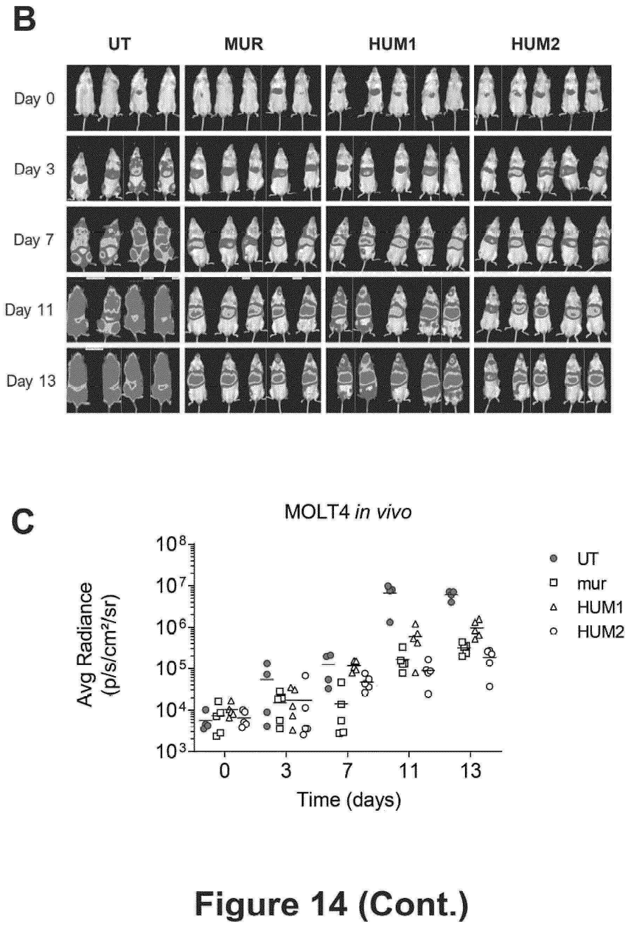

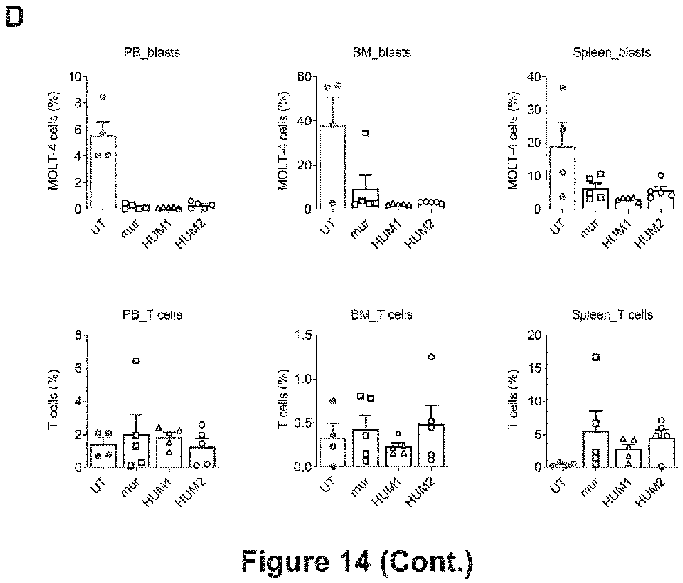

Figure 14. CAR-CCR9 functionality in vivo. A) Schematic showing in vivo testing. NSG mice (n=5) were injected with 1.5×10⁶ (1.5 M) MOLT4Luc cells intravein (iv), and three days later 4×10⁶ (4.5 M) effectorT cells (UT, untransduced; mur, murine CAR-CCR9; humanized 1 CAR-CCR9, HUM1; humanized 2 CAR-CCR9, HUM2) were administered. Disease progression was monitored by bioluminescence bi-weekly, and mice were culled and organs collected and analyzed at day 13 after T cell administration. B) Bioluminescence images monitoring disease progression at indicated timepoints. C) Total radiance quantification at the indicated timepoints (AvgRad, log10 scale). D) Flow cytometry analysis of peripheral blood (PB), bone marrow (BM) and spleen at sacrifice. Percentage of MOLT4 cells (top) and effector T cells (bottom) in the different treatment groups.

### Summary of the invention

In one aspect the present invention relates to a CCR9 targeting moiety that specifically binds to the amino acid sequence of SEQ ID NO:1 (SMEDYVNFN).

In a second aspect the present invention relates to a CCR9 targeting moiety comprising an antibody, F(ab')2, Fab, scFab or scFv, said antibody, F(ab')2, Fab, scFab or scFv comprising
a) a light chain (VL) comprising at least one complementarity determining region (CDR) selected from:
   (i) a CDR comprising the amino acid sequence shown in SEQ ID NO:2 [CDR-L1], or a variant thereof;
   (ii) a CDR comprising the amino acid sequence shown in SEQ ID NO:3 [CDR-L2], or a variant thereof; and
   (iii) a CDR comprising the amino acid sequence shown in SEQ ID NO:4 [CDR-L3], or a variant thereof; and
b) a heavy chain (VH) comprising at least one complementarity determining region (CDR) selected from:
   (i) a CDR comprising the amino acid sequence shown in SEQ ID NO:5 [CDR-H1], or a variant thereof;
   (ii) a CDR comprising the amino acid sequence shown in SEQ ID NO:6 [CDR-H2], or a variant thereof; and
   (iii) a CDR comprising the amino acid sequence shown in SEQ ID NO:7 [CDR-H3], or a variant thereof.

In one embodiment of the first and second aspect of the present invention the CCR9 targeting moiety comprises
a. a VL domain consisting of SEQ ID NO:8 and a VH domain consisting of SEQ ID NO:9; or
b. a VL domain consisting of SEQ ID NO:10 and a VH domain consisting of SEQ ID NO:11; or
c. a VL domain consisting of SEQ ID NO:12 and a VH domain consisting of SEQ ID NO:13.

In one embodiment of the first and second aspect of the present invention the CCR9 targeting moiety is a scFv comprising
a. a VL domain consisting of SEQ ID NO:8 and a VH domain consisting of SEQ ID NO:9; or
b. a VL domain consisting of SEQ ID NO:10 and a VH domain consisting of SEQ ID NO:11; or
c. a VL domain consisting of SEQ ID NO:12 and a VH domain consisting of SEQ ID NO:13.

In one embodiment of present invention the CCR9 targeting moiety is a murine CCR9 targeting moiety.

In one embodiment of present invention the CCR9 targeting moiety is a humanized CCR9 targeting moiety.

In one preferred embodiment of present invention the CCR9 targeting moiety is a humanized targeting moiety comprising the amino acid sequence of SEQ ID NO:16.

In one preferred embodiment of present invention the CCR9 targeting moiety is a humanized targeting moiety comprising the amino acid sequence of SEQ ID NO:17.

In one preferred embodiment of present invention the CCR9 targeting moiety is a murine targeting moiety comprising the amino acid sequence of SEQ ID NO:18.

In a preferred embodiment of present invention the CCR9 targeting moiety consists of the amino acid sequence of SEQ ID NO:16, SEQ ID NO:17 or SEQ ID NO:18.

In a third aspect the present invention relates to a chimeric antigen receptor (CAR) comprising:
a) an extracellular domain comprising a CCR9 targeting moiety according to the first and/or second aspect of present invention;
b) a transmembrane domain; and
c) an intracellular signaling domain.

In one embodiment of the CAR of present invention the transmembrane domain comprises the transmembrane domain of CD28, CD3, CD45, CD4, CD8, CD9, CD16, CD22, CD33, CD37, CD64, CD80, CD86, CD134, CD137, or CD154. In a preferred embodiment the transmembrane domain comprises the transmembrane domain of CD8.

In one embodiment of the CAR of present invention the intracellular signaling domain comprises the intracellular domain of CD3ζ, FcRy, CD3γ, CD3δ, CD3ε, CD5, CD22, CD79a, CD79b or CD66b, preferably of CD3ζ.

In a further embodiment of the CAR of present invention, the CAR further comprises a costimulatory signaling domain, preferably the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276.

In a preferred embodiment of the CAR of present invention, the CAR comprises, preferably consists of the amino acid sequence according to SEQ ID NO:22, SEQ ID NO:23 or SEQ ID NO:24.

In a further embodiment of present invention, the CCR9 targeting moiety, or the CAR further comprise a second targeting-moiety.

In one embodiment said second targeting moiety is selected from a CD3, CD4, CD5, CD7, CD37, CD30, CD33, CD99, CCR7, CDR3, TRBC1/2, or CD1a targeting moiety, preferably wherein said second targeting moiety is a CD1a targeting moiety.

In a preferred embodiment said second targeting moiety is a CD1a targeting moiety. In an even more preferred embodiment, the CD1a targeting moiety is a scFV comprising a VL domain consisting of SEQ ID NO:31 or SEQ ID NO:33 and a VH domain consisting of SEQ ID NO:32 or SEQ ID NO:34.

In a fourth aspect the present invention relates to a nucleic acid encoding the CAR of present invention.

In a fifth aspect the present invention relates to a cell comprising said nucleic acid and/or the CAR of present invention. In a preferred embodiment the cell is a T-cell.

In a sixth aspect the present invention relates to a pharmaceutical composition comprising a plurality of cells of present invention and a pharmaceutically acceptable carrier or diluent.

In a seventh aspect the present invention relates to the cell or the pharmaceutical composition of the invention for use as a medicament.

In an eighth aspect the present invention relates to the cell or the pharmaceutical composition of the invention for use in a method of treating a CCR9-positive cancer, wherein the method comprises administering the cell or composition to a patient in need thereof.

In one embodiment the CCR9-positive cancer is T-cell acute lymphoblastic leukemia, preferably, relapsed/refractory T-cell acute lymphoblastic leukemia.

In a ninth aspect the present invention relates to the cell or the pharmaceutical composition of the invention for use in a method of treating a CD1a-positive cancer, preferably a CD1a and CCR9-positive cancer, wherein the method comprises administering the cell or composition to a patient in need thereof.

In one embodiment the CD1a-positive cancer is cortical T-cell acute lymphoblastic leukemia, preferably, relapsed/refractory cortical T-cell acute lymphoblastic leukemia.

### Definitions

"Administering" or "administration of" a medicament to a patient (and grammatical equivalents of this phrase) refers to direct administration, which may be administration to a patient by a medical professional or may be self-administration, and/or indirect administration, which may be the act of prescribing a drug. E.g., a physician who instructs a patient to self-administer a medicament or provides a patient with a prescription for a drug is administering the drug to the patient.

The term "affibody" refers to a protein that is derived from the Z domain of protein A and that been engineered to bind to a specific target (see Frejd & Kim, 2017. Exp Mol Med. 49(3): e306).

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies. A typical type of antibody comprises at least two heavy chains ("HC") and two light chains ("LC") interconnected by disulfide bonds.

Each "heavy chain" comprises a "heavy chain variable domain" (abbreviated herein as "VH") and a "heavy chain constant domain" (abbreviated herein as "CH"). The heavy chain constant domain typically comprises three constant domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW").

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme).

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

An antibody can be of any the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target (see Skerra, 2008. FEBS J. 275(11):2677-83).

The term "antigen-binding fragment" or "Fab" refers to an antibody fragment comprising one constant and one variable domain of each of the heavy and light chain. A Fab fragment may be obtained by digesting an intact monoclonal antibody with papain.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

The term "CCR9" refers to C-C chemokine receptor (CCR) type 9/CDw199 and is a 7-transmembrane protein chemokine receptor whose unique ligand is CCL25. High expression of CCR9 is associated with poor prognosis or metastasis in different types of solid tumors. Antibodies against CCR9 have recently demonstrated antileukemic effects in preclinical models of T-cell leukemia.

The term "CCR9-targeting moiety" refers to a substance that is able to bind CCR9. Within the context of a CAR, a CCR9-targeting moiety targets T cells to a CCR9-positive cell, preferably a cancer cell. Within the context of a CAR, it is to be understood that the CCR9-targeting moiety is genetically encodable. Exemplary sequence and data related to human CCR9 has been deposited in the UniProtKB database under ID number P51686.

"CCR9-positive" cancer, including a "CCR9-positive" cancerous disease, is one comprising cells, which have CCR9 present at their cell surface. The term "CCR9-positive" also refers to a cancer that produces sufficient levels of CCR9 at the surface of cells thereof, such that a CAR-comprising cell of the present invention has a therapeutic effect, mediated by the binding of the CAR to CCR9. In some embodiments, the CCR9-positive cancer is T-cell acute lymphoblastic leukemia.

The term "CD1a" refers to a non-polymorphic MHC Class 1 related cell surface glycoprotein, expressed in association with β-2-microglobulin. CD1a is expressed by cortical thymocytes, Langerhans cells and by interdigitating cells. CD1a is also expressed by some malignancies of T cell lineage and in Langerhans cell histiocytosis. CD1a is expressed on cortical thymocytes, epidermal Langerhans cells, dendritic cells, on certain T-cell leukemias, and in various other tissues. CD1a is structurally related to the major histocompatibility complex (MHC) proteins and form heterodimers with β-2-microglobulin. Exemplary sequence and data related to human CD1a has been deposited in the UniProtKB database under ID number P06126.

"CD1a-positive" cancer, including a "CD1a-positive" cancerous disease, is one comprising cells, which have CD1a present at their cell surface. The term "CD1a-positive" also refers to a cancer that produces sufficient levels of CD1a at the surface of cells thereof, such that a CAR-comprising cell of the present invention has a therapeutic effect, mediated by the binding of the CAR to CD1a. In some embodiments, the CD1a-positive cancer is cortical T-cell acute lymphoblastic leukemia or Langerhans cell histiocytosis (LCH).

The term "CD1a-targeting moiety" refers to a substance that is able to bind CD1a. Within the context of a CAR, a CD1a-targeting moiety targets T cells to a CD1a-positive cell, preferably a cancer cell. Within the context of a CAR, it is to be understood that the CD1a-targeting moiety is genetically encodable.

The term "chimeric antigen receptor" or "CAR" refers to a synthetic receptor that targets T-cells or other effector cells, such as for example NK cells, gamma delta T cells, or others, to a chosen antigen and reprograms T cell function, metabolism and persistence (see Rivière & Sadelain, 2017. Mol Ther. 25(5):1117-1124). Similarly, the term "CART" refers to a T cell that comprises a CAR.

"Combination therapy", "in combination with" or "in conjunction with" as used herein denotes any form of concurrent, parallel, simultaneous, sequential or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations or unit dosage forms) or separately (e.g., on the same day or on different days and in any order as according to an appropriate dosing protocol for the separate compositions, formulations or unit dosage forms) in a manner and dosing regimen prescribed by a medical caretaker or according to a regulatory agency. In general, each treatment modality will be administered at a dose and/or on a time schedule determined for that treatment modality. Optionally, three or more modalities may be used in a combination therapy. Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

A "complete response" or "complete remission" or "CR" indicates disappearance of all target lesions as defined in the RECIST v1.1 guideline. This does not always mean the cancer has been cured.

The term "costimulatory signaling domain" refers to a signaling moiety that provides to T cells a signal which, in addition to the primary signal provided by for instance the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, 1COS, lymphocyte function-associated antigen-1 (LFA-1), CD2, CD7, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

The term "designed ankyrin repeat proteins" or "DARPin" refers to a protein that is derived from an ankyrin repeat that has been engineered to bind to a specific target (see Plückthun, 2015. Annu Rev Pharmacol Toxicol. 55:489-511).

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats T-ALL, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The term "fynomer" refers to a protein that is derived from the SH3 domain of human Fyn kinase that has been engineered to bind to a specific target (see Bertschinger et al., 2007. Protein Eng Des Sel. 20(2):57-68).

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a CD1a and/or CCR9-positive cancer.

"Infusion" or "infusing" refers to the introduction of a therapeutic agent-containing solution into the body through a vein for therapeutic purposes. Generally, this is achieved via an intravenous bag.

"Intracellular signaling domain" as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen- independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRy, CD3γ, CD3δ, CD3ε, CD5, CD22, CD79a, CD79b, and CD66d.

The term "monobody" refers to a protein that is derived from a fibronectin type II domain that has been engineered to bind to a specific target (see Koide et al., 2013. J Mol Biol. 415(2):393-405).

The term "nanobody" refers to a protein comprising the soluble single antigen-binding V-domain of a heavy chain antibody, preferably a camelid heavy chain antibody (see Bannas et al., 2017. Front Immunol. 8:1603).

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

The term "peptide aptamer" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline and it is published in European Journal of Cancers 45 (2009) 228-247.

The term "repebody" refers to a protein that is derived from a leucine-rich repeat module and that been engineered to bind to a specific target (see Lee et al., 2012. PNAS. 109(9): 3299-3304).

The term "respond favorably" generally refers to causing a beneficial state in a subject. With respect to cancer treatment, the term refers to providing a therapeutic effect on the subject. Positive therapeutic effects in cancer can be measured in a number of ways (See, Weber, 2009. J Nucl Med. 50 Suppl 1:1S-10S). For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

The term "sequence identity" refers to a percentage value obtained when two sequences are compared using a pairwise sequence alignment tool. In the present case, the sequence identity is obtained using the global alignment tool "EMBOSS Needle" using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/ .

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv). Methods of stabilizing scFvs with disulfide bonds are disclosed in Reiter et al., 1996. Nat Biotechnol. 14(10):1239-45.

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrollment to disease progression. TTP is generally measured using the RECIST v1.1 criteria.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

### Detailed description of the invention

The inventors have shown that CCR9 expression varies in healthy tissues and T-ALL samples (Example 1). Specifically, and as can be seen from Figure 1, CCR9 expression is detected in thymocytes and T cells from the small intestine and in human neonatal thymus subpopulations. Low CCR9 expression was found in human peripheral blood and bone marrow from healthy donors with only a slight increase in B-cells (Figure 1B to 1D). In contrast, CCR9 expression is detected in blast cells from T-ALL patients stratified across immunophenotypes/subtypes (Figure 1E & F). In addition, CCR9 expression in T-ALL blasts is similarly detected at diagnosis (Dx) and relapse (Rel) (Figure 1G). CCR9 is therefore a promising target for T-ALL therapy and the inventors have thus embarked on the generation of a CCR9 antibody that can be used in such T-ALL therapy targeting T-ALL cells via CCR9.

Firstly, a murine CCR9 antibody was generated by mouse immunisation with human CCR9 peptides as shown in Figure 2 and Example 2. The immune response was verified by ELISA against the peptides used for the immunisation and screenings at polyclonal stage were conducted and positive clones further tested by FACS (see Figure 3A for a representative analysis). Hybridoma clones were tested by incubating their conditioned media containing the secreted antibodies ("supernatant") with the T-ALL CCR9+ cell line MOLT-4 and the CCR9^{KO} MOLT-4. Only one hybridoma clone tested positive (Clone 115) (Figure 3A) and was thus further tested using a CCR9-negative 300.19 cell line (WT) and its CCR9-overexpressing counterpart (Figure 3B). The isotype of the antibody is IgG1 and ELISA titer and SDS-PAGE quality control analysis are shown in Figure 3C. Finally, the hybridoma #115 was sequenced and a second-generation 4-1BB-based CAR-CCR9 was cloned into a clinically validated pCCL lentivector (Figure 4).

To avoid immunogenic response in humans and to make the CAR T-cells more suitable for use in the clinic, as a next step humanization of the murine scFv was performed using two strategies as summarized in Figure 5 and described in more detail in Example 3. The CAR-CCR9 generated following a sequence-based "strict" method is called hereinafter h1CAR-CCR9 or HUM1. The conservation among the different human sequences for the heavy and light chains is indicated in Figures 8, 9 and 10 and is used to assess the level of conservation among human sequences in non-conserved positions (that are not the CRDs and stem regions) between murine and human sequences. The CAR-CCR9 generated following a sequence-based "relaxed" method is called hereinafter h2CAR-CCR9 or HUM2.

The inventors could show that PBMCs transduced with mCAR-CCR9 and h1CAR-CCR9 and h2CAR-CCR9 could be detected by FACS (Example 4 and Figure12).

They then assessed the capacity of CCR9-CAR T-cells to eliminate target (T) CCR9+ MOLT4 and SupT1 cells (Example 5 and Figure 13). As shown in Figure 13B, murine and HUM2 CCR9 CAR-Ts are equally effective in killing MOLT4 or SupT1 cells but not MV4;11 cells. Killing activity of HUM2 was slightly lower. These results clearly show that humanized CCR9-CAR T-cells are as effective as murine CAR-T cells to promote cell death *in vitro.*

The activity of murine and humanized CCCR9-CAR-Ts *in vivo* using Luc/GFP-expressing MOLT4 T-ALL cells was also evaluated (Example 6, Figure 14). In contrast to the mice receiving UT cells, which showed massive tumor burden by bioluminescence (BLI), those mice given CCR9-CAR-Ts had a lower disease progression until day 13 (Figure 14B). Quantification of BLI by IVIS showed no differences among the murine CAR-CCR9 and the humanized versions (Figure 14C). For the analysis of tumour circulating cells and persistence of the transferred CARTs, the mice were sacrificed at the end of the experiment (day 13). The results show a significant reduction of tumor burden in PB, BM and spleen in mice receiving humanized or murine CAR-Ts related to UT (Figure 14D). Overall, the inventors could show the efficacy of the murine and humanized CCR9-CAR T-cell products, showing a potent and specific antileukemic activity against T-ALL cell lines *in vitro* and potent antileukemic activity *in vivo.*

To assess to which epitope in CCR9 the murine and humanized CCR9-CAR T-cells bind, epitope mapping was performed as further explained in Example 7. A strong binding was observed against the complete antigen peptide MADDYGSESTSSMEDYVNFNF used to generate the 115 antibody. In addition, the purified antibody 115 detected Peptide 3 (SSMEDYVNFN) and Peptide 4 (SMEDYVNFNF) in ELISA with strong signal but no signal was detected with Peptide 1 (MADDYGSEST) and Peptide 2 (GSESTSSMED). As a result, the 9 AA epitope sequence recognized by antibody 115 on the MADDYGSESTSSMEDYVNFNF peptide is SMEDYVNFN.

### CCR9 TARGETING MOIETY

In view of the above results, in one aspect the present invention relates to a CCR9 targeting moiety that specifically binds to the amino acid sequence of SEQ ID NO:1 (SMEDYVNFN).

In a second aspect the present invention relates to a CCR9 targeting moiety comprising an antibody, F(ab')2, Fab, scFab or scFv, said antibody, F(ab')2, Fab, scFab or scFv comprising
a) a light chain domain (VL) comprising at least one complementarity determining region (CDR) selected from:
   (i) a CDR comprising the amino acid sequence shown in SEQ ID NO:2 [LCDR-1], or a variant thereof;
   (ii) a CDR comprising the amino acid sequence shown in SEQ ID NO:3 [LCDR-2], or a variant thereof; and
   (iii) a CDR comprising the amino acid sequence shown in SEQ ID NO:4 [LCDR-3], or a variant thereof; and
b) a heavy chain domain (VH) comprising at least one complementarity determining region (CDR) selected from:
   (i) a CDR comprising the amino acid sequence shown in SEQ ID NO:5 [HCDR-1], or a variant thereof;
   (ii) a CDR comprising the amino acid sequence shown in SEQ ID NO:6 [HCDR-2], or a variant thereof; and
   (iii) a CDR comprising the amino acid sequence shown in SEQ ID NO:7 [HCDR-3], or a variant thereof.

Table 1 below depicts the amino acid sequences SEQ ID NO:2 to SEQ ID NO:7 corresponding to the six CDRs LCDR1-3 and HCDR1-3.

**Table 1: Sequences depicting the six CDRs HCDR1-3 and LCDR1-3**

| | | |
|---|---|---|
| LCDR-1 | SEQ ID NO:2 | QSLVHSNGKTY |
| LCDR-2 | SEQ ID NO:3 | KVS |
| LCDR-3 | SEQ ID NO:4 | AQSTHVWT |
| HCDR-1 | SEQ ID NO:5 | GYSFTDYI |
| HCDR-2 | SEQ ID NO:6 | IDPNNYNT |
| HCDR-3 | SEQ ID NO:7 | ARDVY |

In one embodiment of this second aspect said VL domain comprises the CDRs LCDR1, LCDR2 and LCDR3 and said VH domain comprises the CDRs HCDR1, HCDR2 and HCDR3, wherein:
- LCDR1 comprises, consists, or consists essentially of an amino acid sequence as shown in SEQ ID NO:2, or a sequence which is at least 90%, 91%, 92%, 93%, 94%, 95%, 96%, 97%, 98%, or 100% identical to SEQ ID NO:2;
- LCDR2 comprises, consists, or consists essentially of an amino acid sequence as shown in SEQ ID NO:3, or a sequence which is at least 90%, 91%, 92%, 93%, 94%, 95%, 96%, 97%, 98%, or 100% identical to SEQ ID NO:3;
- LCDR3 comprises, consists, or consists essentially of an amino acid sequence as shown in SEQ ID NO:4, or a sequence which is at least 90%, 91%, 92%, 93%, 94%, 95%, 96%, 97%, 98%, or 100% identical to SEQ ID NO:4;
- HCDR1 comprises, consists, or consists essentially of an amino acid sequence as shown in SEQ ID NO:5, or a sequence which is at least 90%, 91%, 92%, 93%, 94%, 95%, 96%, 97%, 98%, or 100% identical to SEQ ID NO:5;
- HCDR2 comprises, consists, or consists essentially of an amino acid sequence as shown in SEQ ID NO:6, or a sequence which is at least 90%, 91%, 92%, 93%, 94%, 95%, 96%, 97%, 98%, or 100% identical to SEQ ID NO:6; and
- HCDR3 comprises, consists, or consists essentially of an amino acid sequence as shown in SEQ ID NO:7, or a sequence which is at least 90%, 91%, 92%, 93%, 94%, 95%, 96%, 97%, 98%, or 100% identical to SEQ ID NO:7.

In one embodiment the CCR9 targeting moiety is a murine CCR9 targeting moiety.

In one embodiment the CCR9 targeting moiety is a humanized CCR9 targeting moiety.

In some embodiments of present invention, the CCR9-targeting moiety is an antibody, anticalin, repebody, monobody, scFv, Fab, scFab, affibody, fynomer, DARPin, nanobody, or peptide aptamer that specifically binds to CCR9.

In one embodiment of the first and second aspect of the present invention the CCR9 targeting moiety comprises a VL domain consisting of SEQ ID NO:8 and a VH domain consisting of SEQ ID NO:9 (see table 2 below). In a preferred embodiment the CCR9 targeting moiety is a scFv comprising a VL domain consisting of SEQ ID NO:8 and a VH domain consisting of SEQ ID NO:9.

In one embodiment of the first and second aspect of the present invention the CCR9 targeting moiety comprises a VL domain consisting of SEQ ID NO:10 and a VH domain consisting of SEQ ID NO:11 (see table 2 below). In a preferred embodiment the CCR9 targeting moiety is a scFv comprising a VL domain consisting of SEQ ID NO:10 and a VH domain consisting of SEQ ID NO:11.

In one embodiment of the first and second aspect of the present invention the CCR9 targeting moiety comprises a VL domain consisting of SEQ ID NO:12 and a VH domain consisting of SEQ ID NO:13 (see table 2 below). In a preferred embodiment the CCR9 targeting moiety is a scFv comprising a VL domain consisting of SEQ ID NO:12 and a VH domain consisting of SEQ ID NO:13.

**Table 2: Sequences depicting the VL and VH domain of the two humanized CCR9 scFvs (HUM1 and HUM2) and the murine CCR9 scFvs (MUR). The CDRs are highlighted.**

| | | |
|---|---|---|
| VL (HUM1) | SEQ ID NO:8 | DVVMTQTPLSLSVTPGQPASISCRSSQSLVHSNGKTYLQWYLQ KPGQSPKLLIYKVSNRFSGVPDRFSGSGSGTDFTLKISRVEAED VGVYFCAQSTHVWTFGGGTKVEIKRA |
| VH (HUM1) | SEQ ID NO:9 | QVQLKQSGPEVVKPGTSVKVSCTASGYSFTDYIIYWVKQAHGR SLEWMGYIDPNNYNTRYSQKFQGKVTITVDKSSTSAYMELNSL TSDDTAVYYCARDVYWGQGTTVTVSS |
| VL (HUM2) | SEQ ID NO:10 | DVVMTQTPLSLSVTPGQPASISCRSSQSLVHSNGKTYLQWYLQ KPGQSPKLLIYKVSNRFSGVPDRFSGSGSGTDFTLKISRVEAED VGVYFCAQSTHVWTFGGGTKVEIKRA |
| VH (HUM2) | SEQ ID NO:11 | QVQLVQSGAEVKKPGASVKVSCTASGYSFTDYIIYWVKQAHGQ SLEWMGYIDPNNYNTRYSQKFQGKVTITVDKSSTSAYMELNSL TSDDTAVYYCARDVYWGQGTTVTVSS |
| VL (MUR) | SEQ ID NO:12 | DVVMTQTPLSLTVSLGDQASISCRSSQSLVHSNGKTYLQWYLQ KPGQSPKLLIYKVSNRFSGVPDRFSGSGSGTDFTLKISRVEAED LGVYFCAQSTHVWTFGGGTKLEIKRA |
| VH (MUR) | SEQ ID NO:13 | QVQLKQSGPELVKPGTSVRVSCTASGYSFTDYIIYWVKQSHGR SLEWIGYIDPNNYNTRYSQKFKGKATLTVDKSSTSAFMHLNSLT SDDSAVYYCARDVYWGQGTTLTVSS |

In some embodiments, the CCR9-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO:2, LCDR2 consists of SEQ ID NO:3, LCDR3 consists of SEQ ID NO:4, HCDR1 consists of SEQ ID NO:5, HCDR2 consists of SEQ ID NO: 6, and HCDR3 consists of SEQ ID NO:7.

In a preferred embodiment the humanized CCR9 targeting moiety is a scFv comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of an amino acid sequence as shown in SEQ ID NO:2, or a sequence which is at least 90%, 91%, 92%, 93%, 94%, 95%, 96%, 97%, 98%, or 100% identical to SEQ ID NO:2;
- LCDR2 comprises, consists, or consists essentially of an amino acid sequence as shown in SEQ ID NO:3, or a sequence which is at least 90%, 91%, 92%, 93%, 94%, 95%, 96%, 97%, 98%, or 100% identical to SEQ ID NO:3;
- LCDR3 comprises, consists, or consists essentially of an amino acid sequence as shown in SEQ ID NO:4, or a sequence which is at least 90%, 91%, 92%, 93%, 94%, 95%, 96%, 97%, 98%, or 100% identical to SEQ ID NO:4;
- HCDR1 comprises, consists, or consists essentially of an amino acid sequence as shown in SEQ ID NO:5, or a sequence which is at least 90%, 91%, 92%, 93%, 94%, 95%, 96%, 97%, 98%, or 100% identical to SEQ ID NO:5;
- HCDR2 comprises, consists, or consists essentially of an amino acid sequence as shown in SEQ ID NO:6, or a sequence which is at least 90%, 91%, 92%, 93%, 94%, 95%, 96%, 97%, 98%, or 100% identical to SEQ ID NO:6; and
- HCDR3 comprises, consists, or consists essentially of an amino acid sequence as shown in SEQ ID NO:7, or a sequence which is at least 90%, 91%, 92%, 93%, 94%, 95%, 96%, 97%, 98%, or 100% identical to SEQ ID NO:7.

In an even more preferred embodiment, the humanized CCR9 targeting moiety is a scFv comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 consists of an amino acid sequence as shown in SEQ ID NO:2;
- LCDR2 consists of an amino acid sequence as shown in SEQ ID NO:3;
- LCDR3 consists of an amino acid sequence as shown in SEQ ID NO:4;
- HCDR1 consists of an amino acid sequence as shown in SEQ ID NO:5;
- HCDR2 consists of an amino acid sequence as shown in SEQ ID NO:6;
- HCDR3 consists of an amino acid sequence as shown in SEQ ID NO:7.

In a preferred embodiment the CCR9 targeting moiety is an antibody, F(ab')2, Fab, preferably scFv, or scFab comprising a VL domain and a VH domain, wherein the VL domain comprises, consists, or consists essentially of SEQ ID NO:8, or a sequence with at least 90%, 91%, 92%, 93%, 94%, 95%, 96%, 97%, 98%, or 100% sequence identity to SEQ ID NO:8; and wherein the VH domain comprises, consists, or consists essentially of SEQ ID NO:9, or a sequence with at least 90%, 91%, 92%, 93%, 94%, 95%, 96%, 97%, 98%, or 100% sequence identity to SEQ ID NO:9.

In a preferred embodiment the CCR9 targeting moiety is an scFv comprising a VL domain and a VH domain, wherein the VL domain comprises, consists, or consists essentially of SEQ ID NO:8, or a sequence with at least 90%, 91%, 92%, 93%, 94%, 95%, 96%, 97%, 98%, or 100% sequence identity to SEQ ID NO:8; and wherein the VH domain comprises, consists, or consists essentially of SEQ ID NO:9, or a sequence with at least 90%, 91%, 92%, 93%, 94%, 95%, 96%, 97%, 98%, or 100% sequence identity to SEQ ID NO:9.

In another preferred embodiment the CCR9 targeting moiety is an antibody, F(ab')2, Fab, preferably scFv, or scFab comprising a VL domain and a VH domain, wherein the VL domain comprises, consists, or consists essentially of SEQ ID NO:10, or a sequence with at least 90%, 91%, 92%, 93%, 94%, 95%, 96%, 97%, 98%, or 100% sequence identity to SEQ ID NO:10; and wherein the VH domain comprises, consists, or consists essentially of SEQ ID NO:11, or a sequence with at least 90%, 91%, 92%, 93%, 94%, 95%, 96%, 97%, 98%, or 100% sequence identity to SEQ ID NO:11.

In a preferred embodiment the CCR9 targeting moiety is an scFv comprising a VL domain and a VH domain, wherein the VL domain comprises, consists, or consists essentially of SEQ ID NO:10, or a sequence with at least 90%, 91%, 92%, 93%, 94%, 95%, 96%, 97%, 98%, or 100% sequence identity to SEQ ID NO:10; and wherein the VH domain comprises, consists, or consists essentially of SEQ ID NO:11, or a sequence with at least 90%, 91%, 92%, 93%, 94%, 95%, 96%, 97%, 98%, or 100% sequence identity to SEQ ID NO:11.

In another preferred embodiment the CCR9 targeting moiety is an antibody, F(ab')2, Fab, preferably scFv, or scFab comprising a VL domain and a VH domain, wherein the VL domain comprises, consists, or consists essentially of SEQ ID NO:12, or a sequence with at least 90%, 91%, 92%, 93%, 94%, 95%, 96%, 97%, 98%, or 100% sequence identity to SEQ ID NO:12; and wherein the VH domain comprises, consists, or consists essentially of SEQ ID NO:13, or a sequence with at least 90%, 91%, 92%, 93%, 94%, 95%, 96%, 97%, 98%, or 100% sequence identity to SEQ ID NO:13.

In a preferred embodiment the humanized CCR9 targeting moiety is an scFv comprising a VL domain and a VH domain, wherein the VL domain comprises, consists, or consists essentially of SEQ ID NO:12, or a sequence with at least 90%, 91%, 92%, 93%, 94%, 95%, 96%, 97%, 98%, or 100% sequence identity to SEQ ID NO:12; and wherein the VH domain comprises, consists, or consists essentially of SEQ ID NO:13, or a sequence with at least 90%, 91%, 92%, 93%, 94%, 95%, 96%, 97%, 98%, or 100% sequence identity to SEQ ID NO:13.

In one embodiment, the CCR9-targeting moiety is an antibody, scFv, Fab, or scFab comprising a VL domain and VH domain, wherein the VL domain consists of SEQ ID NO:8 and the VH domain consists of SEQ ID NO:9.

In one embodiment, the CCR9-targeting moiety is an antibody, scFv, Fab, or scFab comprising a VL domain and VH domain, wherein the VL domain consists of SEQ ID NO:10 and the VH domain consists of SEQ ID NO:11.

In one embodiment, the CCR9-targeting moiety is an antibody, scFv, Fab, or scFab comprising a VL domain and VH domain, wherein the VL domain consists of SEQ ID NO:12 and the VH domain consists of SEQ ID NO:13.

In a preferred embodiment, the VL and the VH domains comprised in the CCR9-targeting moiety are linked by a peptide linker. In some embodiments, the linker comprises at least 5 amino acids, preferably between 5 and 25, preferably between 10-20 amino acids, most preferably 20 amino acids. Preferably, at least 10, at least 15 of the the amino acids are G. Preferably, the peptide linker comprises, consists or consists essentially of SEQ ID NO:14, or a sequence that has 90%, 91%, 92%, 93%, 94%, 95%, 96%, 97%, 98%, or 100% sequence identity to SEQ ID NO:14 (see table 3 below).

In a preferred embodiment, the CCR9-targeting moiety further comprises a signal peptide that is placed preferably at the N-terminal region of the CCR9-targeting moiety. Preferably, the signal peptide comprises, consists or consists essentially of SEQ ID NO:15, or a sequence that has 90%, 91%, 92%, 93%, 94%, 95%, 96%, 97%, 98%, or 100% sequence identity to SEQ ID NO:15 (see table 3 below).

**Table 3: Sequences depicting the peptide linker and signal peptide according to one embodiment**

| | | |
|---|---|---|
| Peptide linker | SEQ ID NO:14 | GGGGSGGGGSGGGGSGGGGS |
| Signal Peptide | SEQ ID NO:15 | MALPVTGLLLSLGLLLHAARPTG |

Preferably, the CCR9 targeting moiety comprises, consists, or consists essentially of SEQ ID NO: 16, SEQ ID NO: 17 or SEQ ID NO:18, or a sequence that has 90%, 91%, 92%, 93%, 94%, 95%, 96%, 97%, 98%, or 100% sequence identity to SEQ ID NO:16, SEQ ID NO:17, or SEQ ID NO:18. Most preferably, the CCR9 targeting moiety consists of SEQ ID NO:16, SEQ ID NO: 17 or SEQ ID NO:18.

**Table 4: Sequences depicting humanized and murine CCR9 targeting moieties of present invention**

| | | |
|---|---|---|
| Humanized CCR9 targeting moiety (HUM1) | SEQ ID NO:16 | QVQLKQSGPEVVKPGTSVKVSCTASGYSFTDYIIYWVK QAHGRSLEWMGYIDPNNYNTRYSQKFQGKVTITVDKSS TSAYMELNSLTSDDTAVYYCARDVYWGQGTTVTVSSG GGGSGGGGSGGGGSGGGGSDVVMTQTPLSLSVTPGQ PASISCRSSQSLVHSNGKTYLQWYLQKPGQSPKLLIYKV SNRFSGVPDRFSGSGSGTDFTLKISRVEAEDVGVYFCA QSTHVWTFGGGTKVEIKRA |
| Humanized CCR9 targeting moiety (HUM2) | SEQ ID NO:17 | QVQLVQSGAEVKKPGASVKVSCTASGYSFTDYIIYWVK QAHGQSLEWMGYIDPNNYNTRYSQKFQGKVTITVDKSS TSAYMELNSLTSDDTAVYYCARDVYWGQGTTVTVSSG GGGSGGGGSGGGGSGGGGSDVVMTQTPLSLSVTPGQ PASISCRSSQSLVHSNGKTYLQWYLQKPGQSPKLLIYKV SNRFSGVPDRFSGSGSGTDFTLKISRVEAEDVGVYFCA QSTHVWTFGGGTKVEIKRA |
| Murine CCR9 targeting moiety (MUR) | SEQ ID NO:18 | QVQLKQSGPELVKPGTSVRVSCTASGYSFTDYIIYWVKQ SHGRSLEWIGYIDPNNYNTRYSQKFKGKATLTVDKSSTS AFMHLNSLTSDDSAVYYCARDVYWGQGTTLTVSSGGG GSGGGGSGGGGSGGGGSDVVMTQTPLSLTVSLGDQA SISCRSSQSLVHSNGKTYLQWYLQKPGQSPKLLIYKVSN RFSGVPDRFSGSGSGTDFTLKISRVEAEDLGVYFCAQS THVWTFGGGTKLEIKRA |

Preferably, the CCR9 targeting moiety is a scFv that comprises, consists, or consists essentially of SEQ ID NO:16, or a sequence that has 90%, 91%, 92%, 93%, 94%, 95%, 96%, 97%, 98%, or 100% sequence identity to SEQ ID NO:16. Most preferably, the CCR9 targeting moiety is a scFv consisting of SEQ ID NO:16.

Preferably, the CCR9 targeting moiety is a scFv that comprises, consists, or consists essentially of SEQ ID NO:17, or a sequence that has 90%, 91%, 92%, 93%, 94%, 95%, 96%, 97%, 98%, or 100% sequence identity to SEQ ID NO:17. Most preferably, the CCR9 targeting moiety is a scFv consisting of SEQ ID NO:17.

Preferably, the CCR9 targeting moiety is a scFv that comprises, consists, or consists essentially of SEQ ID NO:18, or a sequence that has 90%, 91%, 92%, 93%, 94%, 95%, 96%, 97%, 98%, or 100% sequence identity to SEQ ID NO:18. Most preferably, the CCR9 targeting moiety is a scFv consisting of SEQ ID NO:18.

### CHIMERIC ANTIGEN RECEPTOR (CAR)

The CCR9 targeting moieties as defined above can be part of a chimeric antigen receptor. Thus, in a third aspect the present invention relates to a chimeric antigen receptor (CAR) comprising:
a) an extracellular domain comprising a CCR9 targeting moiety according to the first and/or second aspect of present invention;
b) a transmembrane domain; and
c) an intracellular signaling domain.

The transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, or CD154.

A transmembrane domain may be synthetic or a variant of a naturally occurring transmembrane domain. In some embodiments, synthetic or variant transmembrane domains comprise predominantly hydrophobic residues such as leucine and valine.

In someone embodiment of present invention, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has at least 95%, at least 96%, at least 97%, at least 98%, at least 99% sequence identity on an amino acid level to the respective transmembrane domain.

In one embodiment of present invention, the transmembrane domain comprises the transmembrane domain of CD28, CD3, CD45, CD4, CD8, CD9, CD16, CD22, CD33, CD37, CD64, CD80, CD86, CD134, CD137, or CD154.

In a preferred embodiment the transmembrane domain comprises the transmembrane domain of CD8, or a variant thereof, wherein the variant thereof has at least 95%, at least 96%, at least 97%, at least 98%, at least 99% sequence identity on an amino acid level to the transmembrane domain of CD8 (SEQ ID NO.19).

In some embodiments, the transmembrane domain comprises the transmembrane domain of CD8 (SEQ ID NO.19).

The CARs according to present invention further comprise an intracellular signaling domain. The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In one embodiment, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has at least 95%, at least 96%, at least 97%, at least 98%, at least 99% sequence identity on an amino acid level to respective intracellular domain.

In one embodiment, the intracellular signaling domain comprises the intracellular domain of CD3ζ, FcRy, CD3γ, CD3δ, CD3ε, CD5, CD22, CD79a, CD79b or CD66b.

In a preferred embodiment, the intracellular signaling domain comprises the intracellular domain of CD3ζ or a variant thereof, wherein the variant thereof has at least 95%, at least 96%, at least 97%, at least 98%, at least 99% sequence identity on an amino acid level to the intracellular domain of CD3ζ (SEQ ID NO.20).

In a more preferred embodiment, the intracellular signaling domain comprises or consists of the intracellular domain of CD3ζ (SEQ ID NO.20).

**Table 5: Sequences of Transmembrane and signaling domains**

| | | |
|---|---|---|
| Transmembrane domain CD8 | SEQ ID NO:19 | TTTPAPRPPTPAPTIASQPLSLRPEACRPAAGGAVHTRGLDFA CDIYIWAPLAGTCGVLLLSLVITLYC |
| Intracellular signaling domain CD3ζ | SEQ ID NO:20 | RVKFSRSADAPAYQQGQNQLYNELNLGRREEYDVLDKRRGR DPEMGGKPQRRKNPQEGLYNELQKDKMAEAYSEIGMKGERR RGKGHDGLYQGLSTATKDTYDALHMQALPPR |
| Costimulatory signaling domain CD137 | SEQ ID NO:21 | KRGRKKLLYIFKQPFMRPVQTTQEEDGCSCRFPEEEEGGCEL |

In some embodiments, the CAR may further comprise a costimulatory signaling domain. In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has at least 95%, at least 96%, at least 97%, at least 98%, at least 99% sequence identity on an amino acid level to sequence of the respective intracellular domain.

In a preferred embodiment, the costimulatory signaling domain comprises the intracellular domain of CD137 or a variant thereof, wherein the variant thereof has at least 95%, at least 96%, at least 97%, at least 98%, at least 99% sequence identity on an amino acid level to the intracellular domain of CD137 (SEQ ID NO:21).

In a more preferred embodiment, the costimulatory signaling domain consists of SEQ ID NO:21.

The following full sequence CARs are specifically envisaged:
A CAR comprising
   (i) a scFv comprising a VL domain and VH domain, wherein said VL domain comprises LCDR1 (SEQ ID NO:2), LCDR2 (SEQ ID NO:3) and LCDR3 (SEQ ID NO:4) polypeptides and said VH domain comprises HCDR1 (SEQ ID NO:5), HCDR2 (SEQ ID NO:6) and HCDR3 (SEQ ID NO:7) polypeptides;
   (ii) a transmembrane domain comprising SEQ ID NO:19 or a sequence that has at least 95%, at least 96%, at least 97%, at least 98%, at least 99% sequence identity to SEQ ID NO:19;
   (iii) an intracellular signaling domain comprising SEQ ID NO:20 or a sequence that has at least 95%, at least 96%, at least 97%, at least 98%, at least 99% sequence identity to SEQ ID NO:20; and
   (iv) a costimulatory signaling domain comprising SEQ ID NO:21 or a sequence that has at least 95%, at least 96%, at least 97%, at least 98%, at least 99% sequence identity to SEQ ID NO:21.

A CAR comprising:
(i) a scFv comprising a VL domain and VH domain, wherein said VL domain comprises LCDR1 (SEQ ID NO:2), LCDR2 (SEQ ID NO:3) and LCDR3 (SEQ ID NO:4) polypeptides and said VH domain comprises HCDR1 (SEQ ID NO:5), HCDR2 (SEQ ID NO:6) and HCDR3 (SEQ ID NO:7) polypeptides;
(ii) a transmembrane domain consisting of SEQ ID NO:19;
(iii) an intracellular signaling domain consisting of SEQ ID NO:20; and
(iv) a costimulatory signaling domain consisting of SEQ ID NO:21.

A CAR comprising:
(i) a scFv comprising a VL domain and VH domain, wherein the VL domain consists of SEQ ID NO:8 and the VH domain consists of SEQ ID NO:9;
(ii) a transmembrane domain comprising SEQ ID NO:19 or a sequence that has at least 95%, at least 96%, at least 97%, at least 98%, at least 99% sequence identity to SEQ ID NO:19;
(iii) an intracellular signaling domain comprising SEQ ID NO:20 or a sequence that has at least 95%, at least 96%, at least 97%, at least 98%, at least 99% sequence identity to SEQ ID NO:20; and
(iv) a costimulatory signaling domain comprising SEQ ID NO:21 or a sequence that has at least 95%, at least 96%, at least 97%, at least 98%, at least 99% sequence identity to SEQ ID NO:21.

A CAR comprising:
(i) a scFv comprising a VL domain and VH domain, wherein the VL domain consists of SEQ ID NO:10 and the VH domain consists of SEQ ID NO:11;
(ii) a transmembrane domain comprising SEQ ID NO:19 or a sequence that has at least 95%, at least 96%, at least 97%, at least 98%, at least 99% sequence identity to SEQ ID NO:19;
(iii) an intracellular signaling domain comprising SEQ ID NO:20 or a sequence that has at least 95%, at least 96%, at least 97%, at least 98%, at least 99% sequence identity to SEQ ID NO:20; and
(iv) a costimulatory signaling domain comprising SEQ ID NO:21 or a sequence that has at least 95%, at least 96%, at least 97%, at least 98%, at least 99% sequence identity to SEQ ID NO:21.

A CAR comprising:
(i) a scFv comprising a VL domain and VH domain, wherein the VL domain consists of SEQ ID NO:12 and the VH domain consists of SEQ ID NO:13;
(ii) a transmembrane domain comprising SEQ ID NO:19 or a sequence that has at least 95%, at least 96%, at least 97%, at least 98%, at least 99% sequence identity to SEQ ID NO:19;
(iii) an intracellular signaling domain comprising SEQ ID NO:20 or a sequence that has at least 95%, at least 96%, at least 97%, at least 98%, at least 99% sequence identity to SEQ ID NO:20; and
(iv) a costimulatory signaling domain comprising SEQ ID NO:21 or a sequence that has at least 95%, at least 96%, at least 97%, at least 98%, at least 99% sequence identity to SEQ ID NO:21.

A CAR comprising:
(i) a scFv comprising a VL domain and VH domain, wherein the VL domain consists of SEQ ID NO:8 and the VH domain consists of SEQ ID NO:9;
(ii) a transmembrane domain consisting of SEQ ID NO:19;
(iii) an intracellular signaling domain consisting of SEQ ID NO:20; and
(iv) a costimulatory signaling domain consisting of SEQ ID NO:21.

A CAR comprising:
(i) a scFv comprising a VL domain and VH domain, wherein the VL domain consists of SEQ ID NO:10 and the VH domain consists of SEQ ID NO:11;
(ii) a transmembrane domain consisting of SEQ ID NO:19;
(iii) an intracellular signaling domain consisting of SEQ ID NO:20; and
(iv) a costimulatory signaling domain consisting of SEQ ID NO:21.

A CAR comprising:
(i) a scFv comprising a VL domain and VH domain, wherein the VL domain consists of SEQ ID NO:12 and the VH domain consists of SEQ ID NO:13;
(ii) a transmembrane domain consisting of SEQ ID NO:19;
(iii) an intracellular signaling domain consisting of SEQ ID NO:20; and
(iv) a costimulatory signaling domain consisting of SEQ ID NO:21.

A CAR comprising
(i) a scFv consisting of SEQ ID NO:16;
(ii) a transmembrane domain comprising SEQ ID NO:19 or a sequence that has at least 95%, at least 96%, at least 97%, at least 98%, at least 99% sequence identity to SEQ ID NO:19;
(iii) an intracellular signaling domain comprising SEQ ID NO:20 or a sequence that has at least 95%, at least 96%, at least 97%, at least 98%, at least 99% sequence identity to SEQ ID NO:20; and
(iv) a costimulatory signaling domain comprising SEQ ID NO:21 or a sequence that has at least 95%, at least 96%, at least 97%, at least 98%, at least 99% sequence identity to SEQ ID NO:21.

A CAR comprising
(i) a scFv consisting of SEQ ID NO:17;
(ii) a transmembrane domain comprising SEQ ID NO:19 or a sequence that has at least 95%, at least 96%, at least 97%, at least 98%, at least 99% sequence identity to SEQ ID NO:19;
(iii) an intracellular signaling domain comprising SEQ ID NO:20 or a sequence that has at least 95%, at least 96%, at least 97%, at least 98%, at least 99% sequence identity to SEQ ID NO:20; and
(iv) a costimulatory signaling domain comprising SEQ ID NO:21 or a sequence that has at least 95%, at least 96%, at least 97%, at least 98%, at least 99% sequence identity to SEQ ID NO:21.

A CAR comprising
(i) a scFv consisting of SEQ ID NO:18;
(ii) a transmembrane domain comprising SEQ ID NO:19 or a sequence that has at least 95%, at least 96%, at least 97%, at least 98%, at least 99% sequence identity to SEQ ID NO:19;
(iii) an intracellular signaling domain comprising SEQ ID NO:20 or a sequence that has at least 95%, at least 96%, at least 97%, at least 98%, at least 99% sequence identity to SEQ ID NO:20; and
(iv) a costimulatory signaling domain comprising SEQ ID NO:21 or a sequence that has at least 95%, at least 96%, at least 97%, at least 98%, at least 99% sequence identity to SEQ ID NO:21.

A CAR comprising:
(i) a scFv consisting of SEQ ID NO:16;
(ii) a transmembrane domain consisting of SEQ ID NO:19;
(iii) an intracellular signaling domain consisting of SEQ ID NO:20; and
(iv) a costimulatory signaling domain consisting of SEQ ID NO:21.

A CAR comprising:
(i) a scFv consisting of SEQ ID NO:17;
(ii) a transmembrane domain consisting of SEQ ID NO:19;
(iii) an intracellular signaling domain consisting of SEQ ID NO:20; and
(iv) a costimulatory signaling domain consisting of SEQ ID NO:21.

A CAR comprising:
(i) a scFv consisting of SEQ ID NO:18;
(ii) a transmembrane domain consisting of SEQ ID NO:19;
(iii) an intracellular signaling domain consisting of SEQ ID NO:20; and
a costimulatory signaling domain consisting of SEQ ID NO:21.

A CAR comprising or consisting of SEQ ID NO:22, or a sequence that has at least 95%, at least 96%, at least 97%, at least 98%, at least 99% sequence identity with SEQ ID NO:22.

A CAR comprising or consisting of SEQ ID NO:23, or a sequence that has at least 95%, at least 96%, at least 97%, at least 98%, at least 99% sequence identity with SEQ ID NO:23.

A CAR comprising or consisting of SEQ ID NO:24, or a sequence that has at least 95%, at least 96%, at least 97%, at least 98%, at least 99% sequence identity with SEQ ID NO:24.

**Table 6: Sequences depicting the CAR-CCR9s (h1CAR-CCR9 (HUM1); h2CAR-CCR9 (HUM2); mCAR-CCR9 (MUR)**

| | | |
|---|---|---|
| h1CAR -CCR9 (HUM1) | SEQ ID NO:22 | MALPVTGLLLSLGLLLHAARPTGQVQLKQSGPELVKPGTSVRV SCTASGYSFTDYIIYWVKQSHGRSLEWIGYIDPNNYNTRYSQKF KGKATLTVDKSSTSAFMHLNSLTSDDSAVYYCARDVYWGQGT TLTVSSGGGGSGGGGSGGGGSGGGGSDVVMTQTPLSLTVSL GDQASISCRSSQSLVHSNGKTYLQWYLQKPGQSPKLLIYKVSN RFSGVPDRFSGSGSGTDFTLKISRVEAEDLGVYFCAQSTHVWT FGGGTKLEIKRATTTPAPRPPTPAPTIASQPLSLRPEACRPAAG GAVHTRGLDFACDIYIWAPLAGTCGVLLLSLVITLYCKRGRKKLL YIFKQPFMRPVQTTQEEDGCSCRFPEEEEGGCELRVKFSRSAD APAYQQGQNQLYNELNLGRREEYDVLDKRRGRDPEMGGKPQ RRKNPQEGLYNELQKDKMAEAYSEIGMKGERRRGKGHDGLYQ GLSTATKDTYDALHMQALPPR |
| h2CAR -CCR9 (HUM2) | SEQ ID NO:23 | MALPVTGLLLSLGLLLHAARPTGQVQLKQSGPEVVKPGTSVKV SCTASGYSFTDYIIYWVKQAHGRSLEWMGYIDPNNYNTRYSQK FQGKVTITVDKSSTSAYMELNSLTSDDTAVYYCARDVYWGQGT TVTVSSGGGGSGGGGSGGGGSGGGGSDVVMTQTPLSLSVTP GQPASISCRSSQSLVHSNGKTYLQWYLQKPGQSPKLLIYKVSN RFSGVPDRFSGSGSGTDFTLKISRVEAEDVGVYFCAQSTHVWT FGGGTKVEIKRATTTPAPRPPTPAPTIASQPLSLRPEACRPAAG GAVHTRGLDFACDIYIWAPLAGTCGVLLLSLVITLYCKRGRKKLL YIFKQPFMRPVQTTQEEDGCSCRFPEEEEGGCELRVKFSRSAD APAYQQGQNQLYNELNLGRREEYDVLDKRRGRDPEMGGKPQ RRKNPQEGLYNELQKDKMAEAYSEIGMKGERRRGKGHDGLYQ GLSTATKDTYDALHMQALPPR |
| mCAR-CCR9 (MUR) | SEQ ID NO:24 | MALPVTGLLLSLGLLLHAARPTGQVQLVQSGAEVKKPGASVKV SCTASGYSFTDYIIYWVKQAHGQSLEWMGYIDPNNYNTRYSQK FQGKVTITVDKSSTSAYMELNSLTSDDTAVYYCARDVYWGQGT TVTVSSGGGGSGGGGSGGGGSGGGGSDVVMTQTPLSLSVTP GQPASISCRSSQSLVHSNGKTYLQWYLQKPGQSPKLLIYKVSN RFSGVPDRFSGSGSGTDFTLKISRVEAEDVGVYFCAQSTHVWT FGGGTKVEIKRATTTPAPRPPTPAPTIASQPLSLRPEACRPAAG GAVHTRGLDFACDIYIWAPLAGTCGVLLLSLVITLYCKRGRKKLL YIFKQPFMRPVQTTQEEDGCSCRFPEEEEGGCELRVKFSRSAD APAYQQGQNQLYNELNLGRREEYDVLDKRRGRDPEMGGKPQ RRKNPQEGLYNELQKDKMAEAYSEIGMKGERRRGKGHDGLYQ GLSTATKDTYDALHMQALPPR |

In a preferred embodiment of the CAR of present invention the CAR consists of SEQ ID NO:22.

In a preferred embodiment of the CAR of present invention the CAR consists of SEQ ID NO:23.

In a preferred embodiment of the CAR of present invention the CAR consists of SEQ ID NO:24.

### SECOND TARGETING MOIETY

In a further embodiment of present invention, the humanized CCR9 targeting moiety, or the CAR as described herein above further comprise a second targeting-moiety.

Said second targeting moiety can be a targeting moiety that targets proteins that are overexpressed in T-ALL, such as for example CD3, CD4, CD5, CD7, CD37, CD30, CD33, CD99, CCR7, CDR3, TRBC1/2, or CD1a.⁴⁴

In one embodiment the said second targeting moiety is CD3, CD4, CD5, CD7, CD37, CD30, CD33, CD99, CCR7, CDR3, TRBC1/2, or CD1a targeting moiety.

In a preferred embodiment said second targeting moiety is a humanized CD1a targeting moiety.

In one embodiment the humanized CD1a targeting moiety comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO:25, or a sequence with at least 90%, 91%, 92%, 93%, 94%, 95%, 96%, 97%, 98%, or 100% sequence identity to SEQ ID NO:25;
- LCDR2 comprises, consists, or consists essentially of SEQ ID NO:26, or a sequence with at least 90%, 91%, 92%, 93%, 94%, 95%, 96%, 97%, 98%, or 100% sequence identity to SEQ ID NO:26;
- LCDR3 comprises, consists, or consists essentially of SEQ ID NO:27, or a sequence with at least 90%, 91%, 92%, 93%, 94%, 95%, 96%, 97%, 98%, or 100% sequence identity to SEQ ID NO:27;
- HCDR1 comprises, consists, or consists essentially of SEQ ID NO:28, or a sequence with at least 90%, 91%, 92%, 93%, 94%, 95%, 96%, 97%, 98%, or 100% sequence identity to SEQ ID NO:28;
- HCDR2 comprises, consists, or consists essentially of SEQ ID NO:29, or a sequence with at least 90%, 91%, 92%, 93%, 94%, 95%, 96%, 97%, 98%, or 100% sequence identity to SEQ ID NO:29; and
- HCDR3 comprises, consists, or consists essentially of SEQ ID NO:30, or a sequence with at least 90%, 91%, 92%, 93%, 94%, 95%, 96%, 97%, 98%, or 100% sequence identity to SEQ ID NO:30.

**Table 7: Sequences depicting the six CDRs HCDR1-3 and LCDR1-3 of the CD1a targeting moiety**

| | | |
|---|---|---|
| LCDR-1 | SEQ ID NO:25 | QASQDINKYIA |
| LCDR-2 | SEQ ID NO:26 | IHYTSTL |
| LCDR-3 | SEQ ID NO:27 | LHYDNLPWT |
| HCDR-1 | SEQ ID NO:28 | SGYAFSTYTMH |
| HCDR-2 | SEQ ID NO:29 | YINPNSASTS |
| HCDR-3 | SEQ ID NO:30 | ARGFYTMDY |

In some embodiments, the CD1a-targeting moiety is an antibody, anticalin, repebody, monobody, scFv, Fab, scFab, affibody, fynomer, DARPin, nanobody, or peptide aptamer that specifically binds to CD1a.

In some embodiments, the CD1a-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides as described herein.

In a preferred embodiment the CD1a-targeting moiety is an scFv.

In one embodiment the humanized CD1a targeting moiety is an antibody, F(ab')2, Fab, preferably scFv, or scFab comprising a VL domain and a VH domain, wherein the VL domain comprises, consists, or consists essentially of SEQ ID NO:31, or a sequence with at least 90%, 91%, 92%, 93%, 94%, 95%, 96%, 97%, 98%, or 100% sequence identity to SEQ ID NO:31; and wherein the VH domain comprises, consists, or consists essentially of SEQ ID NO:32, or a sequence with at least 90%, 91%, 92%, 93%, 94%, 95%, 96%, 97%, 98%, or 100% sequence identity to SEQ ID NO:32.

In another embodiment the humanized CD1a targeting moiety is an antibody, F(ab')2, Fab, preferably scFv, or scFab comprising a VL domain and a VH domain, wherein the VL domain comprises, consists, or consists essentially of SEQ ID NO:33, or a sequence with at least 90%, 91%, 92%, 93%, 94%, 95%, 96%, 97%, 98%, or 100% sequence identity to SEQ ID NO: 33; and wherein the VH domain comprises, consists, or consists essentially of SEQ ID NO: 34, or a sequence with at least 90%, 91%, 92%, 93%, 94%, 95%, 96%, 97%, 98%, or 100% sequence identity to SEQ ID NO:34.

**Table 8: Sequences depicting the different VL and VH domains of the CD1a targeting moieties**

| | | |
|---|---|---|
| VL | SEQ ID NO:31 | RDIQMTQSPSSLSASVGDRVTITCQASQDINKYIAWYQFKPGKGP RLLIHYTSTLEPAIPSRFSGSGSGREYTFTISSLQPEDIATYYCLHYD NLPWTFGGGTKVEIKRA |
| VH | SEQ ID NO:32 | QVQLQQSGAEVKKPGASVKVSCKASGYAFSTYTMHWVRQAPGQ GLEWMGYINPNSASTSYAQKFQGRVTMTADKSTNTAYMELSSLR SEDTAVYYCARGFYTMDYWGQGTLVTVSS |
| VL | SEQ ID NO:33 | DIQMTQSPSSVSASVGDRVTISCQASQDINKYIAWYQFKPGKGPR LLIHYTSTLQPAIPSRFSGSGSGREYTLTISSLQPEDFATYYCLHYD NLPWTFGGGTKVEVRRA |
| VH | SEQ ID NO:34 | QVQLVQSGPEVAKPGASVRLSCKASGYAFSTYTMHWVRQAPGQ GLEWMGYINPNSASTSYNQNFKARVTLTADKSTNTAYMHLSSLTS DDTAVYYCGRGFYTMDYWGQGT LVTVSSA |

In a preferred embodiment, the CD1a-targeting moiety is an antibody, F(ab')2, Fab, preferably scFv an antibody, scFv, Fab, or scFab comprising a VL domain and VH domain, wherein the VL domain consists of SEQ ID NO:31 and the VH domain consists of SEQ ID NO:32. Preferably the CD1a-targeting moiety is an scFv.

In another preferred embodiment the CD1a-targeting moiety is an antibody, F(ab')2, Fab, preferably scFv an antibody, scFv, Fab, or scFab comprising a VL domain and VH domain, wherein the VL domain consists of SEQ ID NO:33 and the VH domain consists of SEQ ID NO:34. Preferably the CD1a-targeting moiety is an scFv.

In a preferred embodiment, the VL and the VH domains comprised in the CD1a-targeting moiety are linked by a peptide linker. In some embodiments, the linker comprises at least 5 amino acids, preferably between 5 and 25, preferably between 10-20 amino acids, most preferably 20 amino acids. Preferably, at least 10, at least 15 of the amino acids are G. Preferably, the peptide linker comprises, consists or consists essentially of SEQ ID NO:14, or a sequence that has 90%, 91%, 92%, 93%, 94%, 95%, 96%, 97%, 98%, or 100% sequence identity to SEQ ID NO:14.

In a preferred embodiment, the CD1a-targeting moiety further comprises a signal peptide that is placed preferably at the N-terminal region of the CD1a-targeting moiety. Preferably, the signal peptide comprises, consists or consists essentially of SEQ ID NO:15, or a sequence that has 90%, 91%, 92%, 93%, 94%, 95%, 96%, 97%, 98%, or 100% sequence identity to SEQ ID NO: 15.

Preferably, the CD1a targeting moiety comprises, consists, or consists essentially of SEQ ID NO: 35, or a sequence that has 90%, 91%, 92%, 93%, 94%, 95%, 96%, 97%, 98%, or 100% sequence identity to SEQ ID NO: 35. Most preferably, the CD1a targeting moiety consists of SEQ ID NO:35.

In another preferred embodiment, the CD1a targeting moiety comprises, consists, or consists essentially of SEQ ID NO:36, or a sequence that has 90%, 91%, 92%, 93%, 94%, 95%, 96%, 97%, 98%, or 100% sequence identity to SEQ ID NO:36. Most preferably, the CD1a targeting moiety consists of SEQ ID NO:36.

**Table 9: Sequences depicting humanized CD1a targeting moieties**

| | | |
|---|---|---|
| Humanized CD1a targeting moiety - 1 | SEQ ID NO:35 | QVQLQQSGAEVKKPGASVKVSCKASGYAFSTYTMHWV RQAPGQGLEWMGYINPNSASTSYAQKFQGRVTMTADK STNTAYMELSSLRSEDTAVYYCARGFYTMDYWGQGTL VTVSSGGGGSGGGGSGGGGSGGGGSRDIQMTQSPSS LSASVGDRVTITCQASQDINKYIAWYQFKPGKGPRLLIHY TSTLEPAIPSRFSGSGSGREYTFTISSLQPEDIATYYCLH YDNLPWTFGGGTKVEIKRA |
| Humanized CD1a targeting moiety - 2 | SEQ ID NO:36 | QVQLVQSGPEVAKPGASVRLSCKASGYAFSTYTMHWV RQAPGQGLEWMGYINPNSASTSYNQNFKARVTLTADKS TNTAYMHLSSLTSDDTAVYYCGRGFYTMDYWGQGTLV TVSSAGGGGSGGGGSGGGGSGGGGSDIQMTQSPSSV SASVGDRVTISCQASQDINKYIAWYQFKPGKGPRLLIHY TSTLQPAIPSRFSGSGSGREYTLTISSLQPEDFATYYCLH YDNLPWTFGGGTKVEVRRA |

In one preferred embodiment of present invention the CAR is therefore a CAR comprising a CCR9 targeting moiety as well as a CD1a targeting moiety as described herein. It is to be understood that such CAR encompasses all possible modes of combination of the two targeting moieties in the CAR. For example, a dual CAR is envisaged, preferably with the CCR9 targeting moiety in distal position.

In a further preferred embodiment, the CAR-CCR9 of present invention can be combined with a CAR-CD1a as described herein during treatment.

### NUCLEIC ACIDS

In a fourth aspect the present invention relates to a nucleic acid encoding any one of the targeting moieties of the present invention, including any one of the CARs disclosed above. The nucleic acid sequence that encodes the chimeric receptor links together a number of modular components that can be excised and replaced with other components in order to customize the chimeric receptor for efficient T cell activation and recognition of the target, specifically CCR9 and/or CD1a.

In some embodiments, the nucleic acid is suitable for transducing or transforming a cell. In some embodiments, the nucleic acid is suitable for transducing or transforming a T cell for use in adoptive immunotherapy.

In some embodiments, the nucleic acid is codon optimized for expression in mammalian cells. Codon optimization methods are known in the art.

The nucleic acid of the present invention may be comprised in a γ-retroviral or lentiviral vector which can be used to transduce or transform a T cell. The nucleic acid may also be inserted into a cell through the use of DNA transposons, RNA transfection or genome editing techniques such as TALEN, ZFN and CRISPR/Cas9.

### CELLS

In a fifth aspect the present invention relates to a cell comprising said nucleic acid and/or the CAR of present invention. In a preferred embodiment the cell is a T-cell (referred to as a CART).

In some embodiments, the cell is a naive T cell, memory stem T cell or central memory T cell. It is currently thought that these cells are better suited for adaptive immunotherapy.

In some embodiments, the cell is an autologous T cell. The term "autologous cell" refers to a cell obtained from the same patient that is to be treated using any one of the methods of the present invention. It is noted that flow cytometric analysis of peripheral blood obtained from 40 patients with active T-cell acute lymphoblastic leukemia revealed the presence of normal CD3+CD1a-T-cells in all the patients. Thus, it is entirely possible to treat a patient using an autologous T cell comprising the nucleic acid and/or CAR of the present invention.

In some embodiments, the cell is an allo-tolerant T cell. The term "allo-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art (see section of allogeneic T cells in Rivière & Sadelain, 2017. Mol Ther. 25(5):1117-1124).

In some embodiments the effector T cell is another allogeneic T cell, such as a gamma-delta T cell (referred to as a CAR gamma-delta T cell) prepared form numerous sources, including peripheral blood mononuclear cells (PBMCs), cord blood, and pluripotent stem cells (iPSCs), or it may be an invariant natural killer T (iNKT) cell (referred to as CAR iNKT cell).

In some embodiments the effector cell may be a natural killer (NK) cell (referred to as CAR NK cell) prepared form numerous sources, including peripheral blood mononuclear cells (PBMCs), cord blood, and pluripotent stem cells (iPSCs).

In some embodiments the effector cell may be a macrophage (referred to as CAR macrophage).

In some embodiments, the T cell is a CD3-positive and CCR9 and CD1a-negative T cell.

In some embodiments, the cell is a lymphoid precursor, embryonic stem cell or an induced pluripotent stem cell with the capacity to differentiate into a mature T cell.

### PHARMACEUTICAL COMPOSITION

In a sixth aspect the present invention relates to a pharmaceutical composition comprising a plurality of cells of present invention and a pharmaceutically acceptable carrier or diluent.

A pharmaceutical composition as described herein may also contain other substances. These substances include, but are not limited to, cryoprotectants, surfactants, anti-oxidants, and stabilizing agents. The "term "cryoprotectant" as used herein, includes agents which provide stability to the CARTs against freezing-induced stresses. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum or other human serum components.

In a seventh aspect the present invention relates to the cell or the pharmaceutical composition of the invention for use as a medicament.

### METHODS OF TREATMENT

In an eighth aspect the present invention relates to the cell or the pharmaceutical composition of the invention for use in a method of treating a CCR9-positive cancer, wherein the method comprises administering the cell or composition to a patient in need thereof.

In one embodiment the CCR9-positive cancer is T-cell acute lymphoblastic leukemia, preferably, relapsed/refractory T-cell acute lymphoblastic leukemia.

In a ninth aspect the present invention relates to the cell or the pharmaceutical composition of the invention for use in a method of treating a CD1a-positive cancer, preferably a CD1a and CCR9-positive cancer, wherein the method comprises administering the cell or composition to a patient in need thereof.

In one embodiment the CD1a-positive cancer is cortical T-cell acute lymphoblastic leukemia or Langerhans cell histiocytosis. In some embodiments, the CD1a-positive cancer is relapsed/refractory cortical T-cell acute lymphoblastic leukemia.

In some embodiments, the patient is administered a therapeutically effective amount of cells. In some embodiments, the patient is administered at least 10², 10³, 10⁴, 10⁵, 10⁶, 10⁷, 10⁸, 10⁹ or 10¹⁰ cells. The number of cells will depend upon the ultimate use for which the composition is intended as will the type of cells included therein. For example, if cells that are specific for a particular antigen are desired, then the population will contain greater than 70%, generally greater than 80%, 85% and 90-95% of such cells. For uses provided herein, the cells are generally in a volume of a liter or less, can be 500 ml or less, even 250 ml or less, or 100 ml or less. The clinically relevant number of cells can be apportioned into multiple infusions that cumulatively equal or exceed 10², 10³, 10⁴, 10⁵, 10⁶, 10⁷, 10⁸, 10⁹ or 10¹⁰ cells.

In some embodiments, the cell or pharmaceutical composition is administered intravenously, intraperitoneally, into the bone marrow, into the lymph node, and /or into cerebrospinal fluid.

In some embodiments, the method comprises a combination therapy. In some embodiments, the method comprises further administering an immune checkpoint inhibitor. In a further embodiment, the method comprises further administering an immune checkpoint inhibitor and/or an IAP inhibitor (see WO 2016/054555).

In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely.

In general, the relapse of leukemia can manifest several months or years after the initial remission; however, most relapses occur within two years after the initial treatment. Refractoriness is a term that implies that the patient has no longer responded to at least one therapy strategy after a relapse.

There is a broad consensus in first-line trials for ALL, specifically in adults that a relapse is defined as "detection of more than 5% of blast cells in the bone marrow after a previous achievement of complete remission (CR) or unequivocal demonstration of extramedullary leukemia participation" (see Gökbuget (2017)). The European Working Group on Adult ALL (EWALL) has documented this statement in a consensus recommendation, (see Dohner (2010)) with the additional explanation that "in the case of 5 to 20% of cell blasts at some stage during the intensive treatment phase and / or during regeneration, the evaluation of the bone marrow should be repeated one week later to distinguish among bone marrow relapse and regeneration phenomenon". The cited definition is based on international recommendations for outcome parameters in acute myeloid leukemia (see Cheson (2003) and Chantepie (2013)); that has been extrapolated to several subtypes of ALL, as in the case of T-ALL.

More recently, some trials did not even define the concept of relapse. Therefore, studies with chimeric antigen receptor (CAR) T cells included patients with "measurable disease" and also included patients with haematological relapse (no additional specification) or minimal residual disease (MRE) (see Lee (2015) and Maude (2014) and Gökbuget (2017)).

In some embodiments, the patient to be treated with the method of the present invention is in complete or near-complete remission after treatment with another therapy. It may be preferable desirable to decrease the tumor burden before using the methods of the present invention because since there are several alternative effector T-cells in cases of patients with highly active relapsed/refractory cortical T-cell acute lymphoblastic leukemia. In some embodiments, the patient to be treated with the method of the present invention has previously been treated with another therapy which resulted in a partial response, complete response, stable disease, decrease in progressive disease, reduced time to tumor progression or any combination thereof.

### EXAMPLES

### Example 1: CCR9 expression in healthy tissues and T-ALL samples

CCR9 expression was assessed in healthy tissues and T-ALL samples. For this a Single-cell RNA seq analysis of CCR9 expression was performed in healthy tissues as described in Tabula Sapiens Consortium, Science (2022) (Figure 1A). CCR9 expression is detected in thymocytes and T cells from the small intestine. Furthermore, CCR9 expression was determined in human neonatal thymus subpopulations by FACS (n=4). Low CCR9 expression was found in human peripheral blood (PB, n=18) and bone marrow (BM, n=13) respectively, from adult and pediatric healthy donors, with the exception of B-cells where is slightly increased (Figure 1B to 1D). By contrast, CCR9 is expressed in blast cells from T-ALL patients (n=66) (Figure 1E). CCR9 expression is detected in blasts from T-ALL patients stratified across immunophenotypes/subtypes (n=120) (Figure 1F). In addition, CCR9 expression in T-ALL blasts is similarly detected at diagnosis (Dx) and relapse (Rel) (Figure 1G).

### Example 2: CCR9 antibody generation strategy

Anti-CCR9 antibody-secreting hybridomas were generated by mouse immunisation with human CCR9 peptides with an extra Cys added at C-term fused with KLH to improve immune response and to BSA for ELISA screenings (ProteoGenix) (Figure 2). Given the membrane-bound nature of CCR9, two extracellular sequences/regions were chosen for mouse immunisation. From the extracellular sequence 1, the longest, two different 21-aminoacid long peptides were used (#1 and #2). From the extracellular sequence 2, one 21-aminoacid long peptide was used (#3). Immunization of 5 mice with peptides was achieved by 4-6 injections until obtain an optimal immune response. Immune response was tested after bleeding and titer test by ELISA against the peptides and fusion of spleen cells from best mice was conducted with mouse myeloma cell lines by following standard methods. Screenings at polyclonal stage were conducted by ELISA against the peptides and positive ones were further tested by FACS (Figure 3A shows a representative analysis). Hybridoma clones were tested by incubating their conditioned media containing the secreted antibodies ("supernatant") with the T-ALL CCR9+ cell line MOLT-4 and the CCR9^{KO} MOLT-4. A secondary anti-mouse IgG (H+L) antibody conjugated with Alexa Fluor (A) 647 was used to detect labelled cells. Only one hybridoma clone tested positive by flow cytometry (Clone 115) (Figure 3A). As a summary, a total of 82 hybridoma candidates were tested (Figure 3):
- From MADDYGSESTSSMEDYVNFNF peptide #1: 40 clones were positive by ELISA screening but only clone 115 were tested positive by FACS (Figure 3).
- From MEDYVNFNFTDFYCEKNNVRQ peptide #2: 6 clones were positive by ELISA screening but none of them were positive by FACS.
- From YSQIKEESGIAICTMVYPSDE peptide #3: 36 clones were positive by ELISA but none of them were positive by FACS.

Clone 115 was further tested using the CCR9-negative 300.19 cell line (WT) and its CCR9-overexpressing counterpart (Figure 3B). After selection, hybridoma sub-cloning, isotype determination, high-scale antibody production and purification by ProteinA/G was conducted by standard methods. Isotype is IgG1 and 10 mg of antibody at 2,8 mg/mL was obtained after concentration, dialysis (PBS pH 7,4) and sterilization by membrane filtration. ELISA titer and SDS-PAGE quality control analysis is shown in Figure 3C. The hybridoma #115 was sequenced to obtain the scFv (SEQ number 12 and 13 and Figure 11), using a mouse IgG library primer set (Sánchez-Martínez et al, Blood (2019)), and a second-generation 4-1BB-based CAR-CCR9 was cloned into a clinically validated pCCL lentivector (Figure 4).

### Example 3: Humanization of clone 115

As CAR-CCR9 was of murine origin (mCAR-CCR9), humanization of the murine scFv was performed to avoid immunogenic response in humans and to make the CAR T-cell product more suitable for using in the clinics. Humanization of mCAR-CCR9 has followed 2 strategies as summarized in Figure 5:
1) Sequence-based "Strict" mode
3) Sequence-based "Relaxed" mode

### 1. Sequence-based "Strict" mode:

This approach follows the subsequent protocol:
- Use the sequence of the murine scFv to query the database of IgG (http://www.imgt.org/).
- The search returns a list of human Ig genes ranked by E-value.
- Choose the one with the highest sequence identity to both heavy and light chains (Figures 6A and 7A).
- Besides the CDRs and stems regions look at other changes (non-conserved) and assess whether to change it or not given the conservation among the different human sequences (Figures 8, 9 and 10) and the potential impact at structural/functional level. The latter is assisted by looking at the position and interactions that non-conserved positions make to its environment using the structural model of scFv (for example, close to CDRs or stems regions; residues important for packing; etc).
- Two different strategies to decide how many changes to perform on the non-conserved position were devised: strict and relaxed. The sequence-based "strict mode" tries to make the minimum changes to achieve humanization. In contrast, the "relaxed mode" allows more changes to the human sequence.

The CAR-CCR9 generated following this sequence-based "strict" method is named hereinafter as h1CAR-CCR9 or HUM1. In this case, regarding the heavy chain the query to the IgG database gives the Germline IGHV1-3*01 that shares the highest sequence identity to the heavy chain (60.2%). For the heavy chain, the number of different residues (with different levels of conservation) without considering CDRs is 32 (Figure 6A). Regarding the light chain, we found the Germline IGKV2D-29*02 shares the highest sequence identity to the light chain (82%). For the light chain, the number of different residues without considering CDRs is 13.

The conservation among the different human sequences for the heavy and light chains is indicated in Figures 8, 9 and 10 and is used to assess the level of conservation among human sequences in non-conserved positions (that are not the CRDs and stem regions, indicated in Figures 6, 7 and 10) between murine and human sequences.

According with the above instructions the decision-make process is summarized below. For the heavy chain (see Figure 6B for the indicated positions):
- Position a: K5 murine (V human) which is facing T23 at the Nt stem of H1, probably important for packing function (murine: K-T and human V-K). We decide to keep the murine (K) residue.
- Position b: P9 murine (A human), L11 murine (V human) and V12 murine (K human).
   P9 is located at the start of a beta-sheet with a potential structural role. We decide to keep the murine residue (P).
   L11 is fine to change to V (human) as is fully exposed. It pairs with T109 in the Ct stem of H3 and T109 is both conserved in human/murine. We decide to keep the human (V). V12 is buried in the structure and might be important for packing. The corresponding in human is a K, which is clearly a not conserved substation, hence we decide to keep the murine (V).
- Position c: T16 murine (A human) is fully exposed in a loop but close to V12 (packing loop). We decide to keep the murine (T).
- Position d: R19 murine (K human) is fully exposed in the middle of a beta-sheet. Conservative substitution. We decide to keep the human (K).
- Position e: A stretch with a number of changes: 38-KQSHGRS-44 murine (40-RQAPGQR-44 human) located on a loop between H1 and H2 as well as K38 at interaction distance to D89 D90 at the base of H3 Nt beta.
   K38: contacts with Nt stems of H3: D89 (D murine->E human) and D90 (which is conserved in both). We decide to keep the murine (K).
   S40: Conservative substitution. We decide to keep the human (A).
   H41: Human has a P, which is a very unique amino-acid structure-wise. H might be important for loop conformation in murine (as P in human). We decide to keep the murine (H).
   R43: It may be important for loop conformation. We decide to keep the murine (R)
   S44: Located at VL-VH packing and Nt stem of L3. It faces the 103-GGG. We decide to keep the murine (S).
- Position f: I48 murine (M human): Buried in the core, so might be important in human scaffold: We decide to keep the human (M).
- Position g: R59 murine (K human). It's aligned to an important positions at Nt stem of H2: Y50. We decide to keep the murine (R).
- Position h: composed of stretch 65-KGKA-68 murine (QGRV human) K67 interacts with D90 (conserved), which is related to position K38 in position e (murine preserved). We decide to keep the murine (K67). For the rest amino-acids, we decide to keep the human (Q65 which is fully exposed and V68 which is at starting of a beta sheet.)
- Position i: composed of stretch 70-LTVDK-74 (ITRDT human)
   L70 murine (I human) which is fully buried. We decide to keep the human (I) as might be important for the fold of the human scaffold.
   V72 and K74 are both facing H2 and K74 is facing H1. We decide to keep both murine residues.
- Position j: stretch 76-STS-79 murine (AST human). Part or a loop that stacks against H1. Just after K74 in (i). We decide to keep all murine residues in the region.
- Position k: stretch 80-FMHLN-85 murine (YMELS in human). F80 and H82 are exposed and related to R19 (where human was kept). We decide to keep both human (Y80 and E82). N84 faces a loop containing K67 (important position). We decide to keep the murine (N84).
- Position l: stretch 87-TSDDS91 murine (RSEDT in human). All residues are located in a loop. T87 neighbors D89 which relates to (e) and (h). We decide to keep the murine (T87). D89 seems to be important in murine and given the conserved nature of the substitution to human E, we decide to keep the murine. S91 (murine) is a conserved substitution (T). We decide to keep the human (T91).
- Position m: L108 murine (V human). Buried residues and conserved substitution. We decide to keep the human (V).

And for the light chain (Figure 7B):
- Position a: V2 murine (I human) at Nt is sandwiched between L1 and L3. We decide to keep the murine (V).
- Position b: composed of stretch 12-TVSLGDQ-18 murine (SVTPGQP human) are all in a exposed loop just before the Nt beta of L1.
   T12 is S in human is a conservative substitution. Keep the human.
   S14 is T in human is a conservative substitution. Keep the human.
   L15, Q18 are both P in human and might play a structural in human scaffold without obvious implication to function of murine. Keep the human.
   D17 is Q in human is a conservative substitution. Keep the human
- Position c: K50 murine (Q human). It is s located at the base of Nt beta of L2 and close to the interface with H3. We decide to keep the murine (K).
- Position d: L88 murine (V human). It is partially exposed with conserved residues around V in human. A conservative substitution. We decide to keep the human (V).
- Position e: F92 is a Y in human. Might be important for interface packing and it is close to position h in heavy chain. We decide to keep the murine (F).
- Position f: L108 murine (V human) is a fully buried aminoacid and could be important for structure of the human scaffold. We decide to keep the human (V).

Figure 8 summarizes the above indicted changes and the proposed humanized sequence. According with this methodology, the final selected sequence in the 'strict mode' is the next:
Heavy chain 
QVQLKQSGPEVVKPGTSVKVSCTASGYSFTDYIIYWVKQAHGRSLEWMGYIDPNNYNTRYSQ
KFQGKVTITVDKSSTSAYMELNSLTSDDTAVYYCARDVYWGQGTTVTVSS
Light chain

DVVMTQTPLSLSVTPGQPASISCRSSQSLVHSNGKTYLQWYLQKPGQSPKLLIYKVSNRFSGV
PDRFSGSGSGTDFTLKISRVEAEDVGVYFCAQSTHVWTFGGGTKVEIKRA

### 2. Sequence-based "Relaxed" mode

A different humanized sequence was selected allowing more amino acid changes in the human sequence with respect to the murine sequence. In Figure 10 are indicated both the "strict" and "relaxed" sequences together with the murine original sequence. The proposed "relaxed" sequence is indicated below:
Heavy chain 
QVQLVQSGAEVKKPGASVKVSCTASGYSFTDYIIYWVKQAHGQSLEWMGYIDPNNYNTRYSQ
KFQGKVTITVDKSSTSAYMELNSLTSDDTAVYYCARDVYWGQGTTVTVSS
Light chain

DVVMTQTPLSLSVTPGQPASISCRSSQSLVHSNGKTYLQWYLQKPGQSPKLLIYKVSNRFSGV
PDRFSGSGSGTDFTLKISRVEAEDVGVYFCAQSTHVWTFGGGTKVEIKRA

### Example 4: CAR-CCR9 detection in transduced T cells

PBMCs transduced with mCAR-CCR9 (mur) and h1CAR-CCR9 (HUM1) and h2CAR-CCR9 (HUM2) could be detected by FACS. Figure 12 shows a representative flow cytometry plots showing CAR construct detection in transduced T cells (GFP+) and anti-scFv. Histogram analysis of transduced cells show the murine (mur) and HUM2 CAR constructs are detectable with anti-F(ab')2 antibodies (anti-scFv).

### Example 5: CAR-CCR9 cell line panel cytotoxicity

The capacity of CAR-CCR9 T-cells to eliminate target (T) CCR9+ MOLT4 and SupT1 cells was analyzed (Figure 13A). For this purpose, target cells (100.000 cells per condition) were labelled with 3 µM eFluor 670 and incubated with UT PBMCs and indicated CCR9-CAR-Ts at different effector (E):Target (T) ratios during 24 or 48 hours. The CCR9- MB4;11 cell line was employed as a control of specificity (Figure 13A). Cell viability was monitored by eFluor labelling of target cells and detection by FACS and after incubation with 7-amino actinomycin D (7-ADD) at each time point and E:T ratio. Percentage of cell populations were calculated by FACSDiva software analysis. Fraction of non-apoptotic cells were further quantified after incubation with Annexin V protein. The fraction of eFluor+/AnnexinV-/7ADD- was considered as non-apoptotic/death cells (Figure 13B) and analyzed with n=5 different donors. As shown in Figure 13B, murine and HUM2 CAR-CCR9-Ts are equally effective in killing MOLT4 or SupT1 cells but not MV4;11 cells. Killing activity of HUM1 was slightly lower. Altogether, these results clearly show that humanized CCR9--CAR T cells are as effective as murine CAR-T cells to promote cell death *in vitro.*

### Example 6: CAR-CCR9 functionality in vivo

The activity of murine and humanized CCR9-CAR-Ts in vivo using Luc/GFP-expressing MOLT4 T-ALL cells was also evaluated (Figure 14). Immunodeficient mouse (NOD scid gamma) NSG mice were transplanted with 1,5×10⁶ Luc-expressing MOLT4 cells 3 days before i.v. infusion of either 4×10⁶ CCR9-CAR-Ts or UT PBMCs, and leukemia establishment was followed up bi-weekly by using bioluminescence (BLI) quantification (Figure 14A). To measure BLI, mice were given 150 mg/kg of D-luciferin intraperitoneally which, when interacting with luciferase, emitted a bioluminescent signal proportional to the number of tumor cells. This signal is measured on a Xenogen IVIS 50 Imaging System (Perkin Elmer). The tumour burden was monitored at day 0 (day of CAR infusion), 3, 7, 11 and 13. Living Image software was used to visualise and calculate total luminescence as average radiance quantification (p/sec/cm2/sr). In contrast to the mice receiving UT cells, which showed massive tumor burden by BLI, those mice given CCR9-CAR-Ts had a lower disease progression until day 13 (Figure 14B). Quantification of BLI by IVIS showed no differences among the murine CAR-CCR9 and the humanized versions (Figure 14C).

For the analysis of tumour circulating cells and persistence of the transferred CARTs, the mice were sacrificed at the end of the experiment (day 13) and circulating peripheral blood (PB), hindlimbs and spleen were taken. BM cells were isolated by flushing the hindlimbs with PBS containing 2%FBS. Spleen cells were isolated by mincing the spleen by crushing with a sterile syringe piston and passing the cell suspension through a 70 µm cell strainer. For the FACS analysis, 100 µl of PB, BM and spleen cell suspension was stained with PBS/2%FBS containing fluorochrome ligated antibodies for the following markers: human leucocyte antigen (HLA)-ABC, CD45, CD3, CCR9, CD1a, CD38, for 30 min at 4 degrees. 1 ml of BD FACSTM fixing-lysis buffer (BD Biosciences) was added to each sample to eliminate erythrocytes. Samples were run in a FACSCantoTM-II flow cytometer. The % of blasts were determined as HLA-ABC+ CD45+ CD34+ CD3- and confirmed to be CD1a+ (and CCR9+). The results show a significant reduction of tumor burden in PB, BM and spleen in mice receiving CCR9 humanized or murine CAR-Ts related to UT (Figure 14D). Human CAR-T cells (HLA-ABC+ CD45+ CD3+) can be detected by FACS in the different compartments (Figure 14D). Overall, the above data demonstrates the efficacy of the murine and humanized CCR9-CAR T-cell products, showing a potent and specific antileukemic activity against T-ALL cell lines *in vitro* and potent antileukemic activity *in vivo.*

### Example 7: Epitope Mapping

In order to assess to which epitope in CCR9 clone 115 binds, an epitope mapping was performed. The antibody for the mapping was produced as described in example 2. Peptides were synthesized with a purity>98% with an extra C-terminal Cys for BSA conjugation. As a positive control the peptide used for mice immunization was used MADDYGSESTSSMEDYVNFNF-C. Then, the next battery of peptides were synthesized covering the whole sequence from the N-terminal to the C-terminal part:
- Peptide 1: MADDYGSEST-C
- Peptide 2: GSESTSSMED-C
- Peptide 3: SSMEDYVNFN-C
- Peptide 4: SMEDYVNFNF-C

Binding of peptides to 115 antibodies were tested by ELISA titration. In brief, plates with each non-conjugated peptide (50µg/mL) or BSA-conjugated peptide (5µg/mL) were coated in 0.58M carbonate-bicarbonate buffer pH9.5, 100µL/well, at 4°C O/N. After washing with PBS + Tween-20 0.05% v/v (0.05%PBST) and blocking with 3%milk-PBS, peptide coated wells were incubated with 115 purified antibodies at concentration ranging from 0 to 1µg/mL in PBS, washed and incubated with a secondary antibody (goat anti-mouse IgG-HRP). After washing, wells were incubated with the 3, 3',5 ,5'-Tetramethylbenzidine (TMB) HRP substrate and reactions were stopped with 2M HCl and OD450 was recorded. Table 1 shows the obtained results.

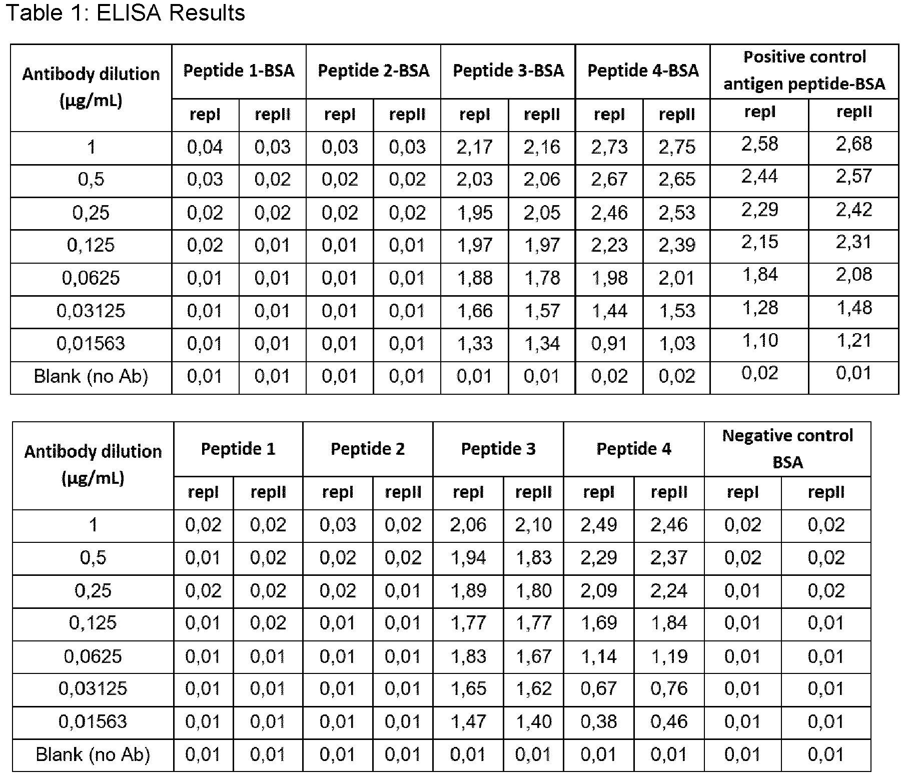

The measured OD shows a strong binding against the complete antigen peptide MADDYGSESTSSMEDYVNFNF, used to generate the 115 antibody. In addition, the purified antibody 115 detect Peptide 3 (SSMEDYVNFN) and Peptide 4 (SMEDYVNFNF) in ELISA with strong signal but no signal was detected with Peptide 1 (MADDYGSEST) and Peptide 2 (GSESTSSMED). As a result, the 9 AA epitope sequence recognized by antibody 115 on the MADDYGSESTSSMEDYVNFNF peptide is highlighted in the full peptide antigen sequence: MADDYGSESTS**SMEDYVNFN**F.

### Bibliography

1. Qasim W, Thrasher AJ. Progress and prospects for engineered T cell therapies. Br. J. Haematol. 2014;166(6):818-829.
2. Goldsmith SR, Slade M, Dipersio JF, et al. Donor-lymphocyte infusion following haploidentical hematopoietic cell transplantation with peripheral blood stem cell grafts and PTCy. Bone Marrow Transplant. 2017;52(12):1623-1628.
3. Barrett DM, Grupp SA, June CH. Chimeric Antigen Receptor- and TCR-Modified T Cells Enter Main Street and Wall Street. J. Immunol. 2015;195(3):755-761.
4. Milone MC, Fish JD, Carpenito C, et al. Chimeric receptors containing CD137 signal transduction domains mediate enhanced survival of T cells and increased antileukemic efficacy in vivo. Mol. Ther. 2009;17(8):1453-1464.
5. Park JH, Brentjens RJ. Are all chimeric antigen receptors created equal? J. Clin. Oncol. 2015;33(6):651-653.
6. Humphries C. Honing that killer instinct. Cancer Immunother. 2013;6-8.
7. Maude SL, Frey N, Shaw PA, et al. Chimeric Antigen Receptor T Cells for Sustained Remissions in Leukemia. N. Engl. J. Med. 2014;371(16):1507-1517.
8. Kristina Karrman and Bertil Johansson. Pediatric T-Cell Acute Lymphoblastic Leukemia. Genes. Chromosomes Cancer. 2017;56(January):89-116.
9. Litzow MR, Ferrando AA. How I treat T-cell acute lymphoblastic leukemia in adults. Blood. 2015;126(7):833-841.
10. Stephen P. Hunger and CGM. Acute lymphoblastic leukemia in children. N. Engl. J. Med. 2015;373(16):1541-1552.
11. Sutton R, Shaw PJ, Venn NC, et al. Persistent MRD before and after allogeneic BMT predicts relapse in children with acute lymphoblastic leukaemia. Br. J. Haematol. 2015; 168(3):395-404.
12. Gomes-Silva D, Srinivasan M, Sharma S, et al. CD7-edited T cells expressing a CD7-specific CAR for the therapy of T-cell malignancies. Blood. 2017;130(3):285-296.
13. Mamonkin M, Rouce RH, Tashiro H, Brenner MK. A T-cell-directed chimeric antigen receptor for the selective treatment of T-cell malignancies. Blood. 2015;126(8):983-992.
14. Png YT, Vinanica N, Kamiya T, et al. Blockade of CD7 expression in T cells for effective chimeric antigen receptor targeting of T-cell malignancies. Blood Adv. 2017;1(25):2348-2360.
15. Cooper ML, Choi J, Staser K, et al. An "off-the-shelf" fratricide-resistant CAR-T for the treatment of T cell hematologic malignancies. Leukemia. 2018;32(9):1970-1983.
16. Rasaiyaah J, Georgiadis C, Preece R, Mock U, Qasim W. TCRαβ/CD3 disruption enables CD3-specific antileukemic T cell immunotherapy. JCI insight. 2018;3(13):.
17. Dai Z, Mu W, Zhao Y, et al. T cells expressing CD5/CD7 bispecific chimeric antigen receptors with fully human heavy-chain-only domains mitigate tumor antigen escape. Signal Transduct. Target. Ther. 2022;7(1):.
18. Dai Z, Mu W, Zhao Y, et al. The rational development of CD5-targeting biepitopic CARs with fully human heavy-chain-only antigen recognition domains. Mol. Ther. 2021;29(9):2707-2722.
19. Zhang M, Chen D, Fu X, et al. Autologous Nanobody-Derived Fratricide-Resistant CD7-CAR T-cell Therapy for Patients with Relapsed and Refractory T-cell Acute Lymphoblastic Leukemia/Lymphoma. Clin. Cancer Res. 2022;OF1-OF14.
20. Pan J, Tan Y, Wang G, et al. Donor-Derived CD7 Chimeric Antigen Receptor T Cells for T-Cell Acute Lymphoblastic Leukemia: First-in-Human, Phase I Trial. J. Clin. Oncol. 2021;39(30):3340-3351.
21. Feng J, Xu H, Cinquina A, et al. Treatment of Aggressive T Cell Lymphoblastic Lymphoma/leukemia Using Anti-CD5 CAR T Cells. Stem Cell Rev. Reports. 2021;17(2):652-661.
22. Qasim W, Zhan H, Samarasinghe S, et al. Molecular remission of infant B-ALL after infusion of universal TALEN gene-edited CAR T cells. Sci. Transl. Med. 2017;9(374):1-9.
23. Qasim W. Genome editing of therapeutic T cells. Gene Genome Ed. 2021;2(January):100010.
24. Georgiadis C, Rasaiyaah J, Gkazi SA, et al. Base-edited CAR T cells for combinational therapy against T cell malignancies. Leukemia. 2021;35(12):3466-3481.
25. Li S, Wang X, Yuan Z, et al. Eradication of T-ALL cells by CD7-targeted universal CAR-T cells and initial test of ruxolitinib-based CRS management. Clin. Cancer Res. 2021;27(5): 1242-1246.
26. Bechan GI, Lee DW, Zajonc DM, et al. Phage display generation of a novel human anti-CD1A monoclonal antibody with potent cytolytic activity. Br. J. Haematol. 2012;159(3):299-310.
27. Silva EAC, Nowak W, Tessone L, et al. CD207+CD1a+ cells circulate in pediatric patients with active Langerhans cell histiocytosis. Blood. 2017;130(17):1898-1902.
28. Sánchez-Martínez D, Baroni ML, Gutierrez-Agüera F, et al. Fratricide-resistant CD1a-specific CAR T cells for the treatment of cortical T-cell acute lymphoblastic leukemia. Blood. 2019;133(21):2291-2304.
29. Zabel BA, Agace WW, Campbell JJ, et al. Human G Protein - coupled Receptor GPR-9-6 / CC Chemokine Receptor 9 Is Selectively Expressed on Intestinal Chemokine - mediated Chemotaxis. Cell. 1999;190(9):1241-55.
30. Amersi FF, Terando AM, Goto Y, et al. Activation of CCR9/CCL25 in cutaneous melanoma mediates preferential metastasis to the small intestine. Clin. Cancer Res. 2008; 14(3):638-645.
31. Singh S, Singh UP, Stiles JK, Grizzle WE, Lillard JW. Expression and functional role of CCR9 in prostate cancer cell migration and invasion. Clin. Cancer Res. 2004;10(24):8743-8750.
32. Zhang Z, Qin C, Wu Y, et al. CCR9 as a prognostic marker and therapeutic target in hepatocellular carcinoma. Oncol. Rep. 2014;31(4):1629-1636.
33. Kong H, Yu W, Chen Z, et al. CCR9 initiates epithelial-mesenchymal transition by activating Wnt/p-catenin pathways to promote osteosarcoma metastasis. Cancer Cell Int. 2021;21(1):1-16.
34. Somovilla-Crespo B, Monzón MTM, Vela M, et al. 92R Monoclonal antibody inhibits human CCR9+ leukemia cells growth in NSG mice xenografts. Front. Immunol. 2018;9(JAN):
35. Maciocia PM, Wawrzyniecka PA, Maciocia NC, et al. Anti-CCR9 Chimeric Antigen Receptor T cells for T Cell Acute Lymphoblastic Leukemia. Blood. 2022;
36. Jones RC, Karkanias J, Krasnow MA, et al. The Tabula Sapiens: A multiple-organ, single-cell transcriptomic atlas of humans. Science (80-. ). 2022;376(6594):.
37. Uehara S, Grinberg A, Farber JM, Love PE. A Role for CCR9 in T Lymphocyte Development and Migration. J. Immunol. 2002;168(6):2811-2819.
38. Torres M, Fernández-Fuentes N, Fiser A, Casadevall A. The immunoglobulin heavy chain constant region affects kinetic and thermodynamic parameters of antibody variable region interactions with antigen. J. Biol. Chem. 2007;282(18):13917-13927.
39. Mirela-Bota P, Aguirre-Plans J, Meseguer A, et al. Galaxy InteractoMIX: An Integrated Computational Platform for the Study of Protein-Protein Interaction Data. J. Mol. Biol. 2021;433(11):166656.
40. Baroni ML, Sanchez Martinez D, Gutierrez Aguera F, et al. 41BB-based and CD28-based CD123-redirected T-cells ablate human normal hematopoiesis in vivo. J. Immunother. cancer. 2020;8(1):.
41. Velasco-Hernandez T, Zanetti SR, Roca-Ho H, et al. Efficient elimination of primary B-ALL cells in vitro and in vivo using a novel 4-1BB-based CAR targeting a membrane-distal CD22 epitope. J. Immunother. Cancer. 2020;8(2):1-11.
42. García-Peydró M, Fuentes P, Mosquera M, et al. The NOTCH1/CD44 axis drives pathogenesis in a T cell acute lymphoblastic leukemia model. J. Clin. Invest. 2018; 128(7):2802-2818.
43. Juan M, Delgado J, Calvo G, Trias E, Urbano-Ispizua Á. Is Hospital Exemption an Alternative or a Bridge to European Medicines Agency for Developing Academic Chimeric Antigen Receptor T-Cell in Europe? Our Experience with ARI-0001. Hum. Gene Ther. 2021; 32(19-20): 1004-1007.
44. Ren, A.; Tong, X.; Xu, N.; Zhang, T.; Zhou, F.; Zhu, H. CAR T-Cell Immunotherapy Treating T-ALL: Challenges and Opportunities. Vaccines 2023, 11, 165. https://doi.org/10.3390/vaccines 11010165).

## Claims

1. A CCR9 targeting moiety that specifically binds to the amino acid sequence of SEQ ID NO:1 (SMEDYVNFN).

2. A CCR9 targeting moiety comprising an antibody, F(ab')2, Fab, scFab or scFv, said antibody, F(ab')2, Fab, scFab or scFv comprising
a. a light chain domain (VL) comprising at least one complementarity determining region (CDR) selected from
(i) a CDR comprising the amino acid sequence shown in SEQ ID NO:2 [LCDR-1], or a variant thereof;
(ii) a CDR comprising the amino acid sequence shown in SEQ ID NO:3 [LCDR-2], or a variant thereof; and
(iii) a CDR comprising the amino acid sequence shown in SEQ ID NO:4 [LCDR-3], or a variant thereof; and
b. a heavy chain domain (VH) comprising at least one complementarity determining region (CDR) selected from
(i) a CDR comprising the amino acid sequence shown in SEQ ID NO:5 [HCDR-1], or a variant thereof;
(ii) a CDR comprising the amino acid sequence shown in SEQ ID NO:6 [HCDR-2], or a variant thereof; and
(iii) a CDR comprising the amino acid sequence shown in SEQ ID NO:7 [HCDR-3], or a variant thereof.

3. The CCR9 targeting moiety of claims 1 or 2, comprising
a. a VL domain consisting of SEQ ID NO:8 and a VH domain consisting of SEQ ID NO:9; or
b. a VL domain consisting of SEQ ID NO:10 and a VH domain consisting of SEQ ID NO:11; or
c. a VL domain consisting of SEQ ID NO:12 and a VH domain consisting of SEQ ID NO:13.

4. The CCR9 targeting moiety according to any one of the preceding claims, wherein the CCR9 targeting moiety is a scFv comprising
a. a VL domain consisting of SEQ ID NO:8 and a VH domain consisting of SEQ ID NO:9; or
b. a VL domain consisting of SEQ ID NO:10 and a VH domain consisting of SEQ ID NO:11;
or
c. a VL domain consisting of SEQ ID NO:12 and a VH domain consisting of SEQ ID NO:13.

5. The CCR9 targeting moiety according to any one of the preceding claims, wherein the CCR9 targeting moiety comprises the amino acid sequence of SEQ ID NO:16, SEQ ID NO:17 or SEQ ID NO:18.

6. A chimeric antigen receptor (CAR) comprising:
a. an extracellular domain comprising a CCR9 targeting moiety according to any one of claims 1 to 5;
b. a transmembrane domain; and
c. an intracellular signaling domain.

7. The CAR according to claim 6, wherein
a. the transmembrane domain comprises the transmembrane domain of CD28, CD3, CD45, CD4, CD8, CD9, CD16, CD22, CD33, CD37, CD64, CD80, CD86, CD134, CD137, or CD154, preferably wherein the transmembrane domain comprises the transmembrane domain of CD8; and/or
b. the intracellular signaling domain comprises the intracellular domain of CD3ζ, FcRy, CD3γ, CD3δ, CD3ε, CD5, CD22, CD79a, CD79b or CD66b, preferably of CD3ζ; and/or
c. the CAR further comprises a costimulatory signaling domain, preferably the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276.

8. The CAR according to any one of claims 6 to 7 consisting of the amino acid sequence according to SEQ ID NO:22, SEQ ID NO:23 or SEQ ID NO:24.

9. The CCR9 targeting moiety or CAR of any one of the preceding claims further comprising a second targeting-moiety, wherein said second targeting moiety is selected from a CD3, CD4, CD5, CD7, CD37, CD30, CD33, CD99, CCR7, CDR3, TRBC1/2, or CD1a targeting moiety, preferably wherein said second targeting moiety is a CD1a targeting moiety.

10. The CCR9 targeting moiety or CAR of any one of the preceding claims, wherein the CD1a targeting moiety is a scFV comprising a VL domain consisting of SEQ ID NO:31 or SEQ ID NO:33 and a VH domain consisting of SEQ ID NO:32 or SEQ ID NO:34.

11. A nucleic acid encoding the CAR according to any one of claims 6-10.

12. A cell comprising the nucleic acid according to claim 11 and/or the CAR according to any one of claims 6-10, preferably wherein the cell is a T-cell.

13. A pharmaceutical composition comprising a plurality of cells according to claim 12 and a pharmaceutically acceptable carrier or diluent.

14. The cell according to claim 12 or the pharmaceutical composition according to claim 13 for use in a method of treating a CCR9-positive cancer, wherein the method comprises administering the cell or composition to a patient in need thereof, preferably wherein the CCR9-positive cancer is T-cell acute lymphoblastic leukemia, more preferably relapsed/refractory T-cell acute lymphoblastic leukemia.

15. The cell according to claim 12 or the pharmaceutical composition according to claim 13 for use in a method of treating a CD1a-positive cancer, preferably a CD1a and CCR9-positive cancer, wherein the method comprises administering the cell or composition to a patient in need thereof, preferably wherein the CD1a-positive cancer is cortical T-cell acute lymphoblastic leukemia, more preferably relapsed/refractory cortical T-cell acute lymphoblastic leukemia.
